# EUROPEAN PATENT APPLICATION

(11) **EP 4 049 676 A1**
(43) Date of publication of application: **31.08.2022**
(21) Application number: 20879885.0
(22) Date of filing: 23.10.2020
(51) Int. Cl.: A61K 39/395, C07K 1/22, C07K 7/08, C07K 16/00, C07K 19/00, A61K 47/64, A61K 51/10, C12N 11/00

(54) **METHOD FOR PRODUCING MONOVALENT CCAP PRODUCT**

(30) Priority: 24.10.2019 JP 2019193830
(71) Applicant: Kagoshima University, Kagoshima-shi, Kagoshima 890-8580 (JP)
(72) Inventor: ITO, Yuji, Kagoshima-shi, Kagoshima 890-8580 (JP); TAKAHASHI, Nobuaki, Tokyo 100-0004 (JP); NAKANO, Ryosuke, Tokyo 100-0004 (JP); MAEDA, Sayaka, Tokyo 100-0004 (JP)
(74) Representative: v. Bezold & Partner Patentanwälte - PartG mbB
(86) International application number: PCT/JP2020/039978
(87) International publication number: WO 2021/080008

(57) **Abstract**

An objective is to provide an Fc-modified antibody or the like having a long serum half-life according to a CCAP method, more specifically, an antibody or the like where IgBP is bound to only one site, based on findings of the inventors. Provided is an objective antibody or the like by purification and production of an Fc-modified antibody or the like with a column bound to an Fc region of an antibody. Specifically, an antibody where only one of two binding sites of Fc is selectively modified is provided by allowing an IgBP-bound antibody produced by a CCAP method to adsorb to a carrier of an IgBP-immobilized column, or forming a state where only one Fc of an antibody is bound to an IgBP binding column and then adding a peptide reagent for CCAP to the column to perform a reaction of a CCAP method in the column.

## Description

### Cross Reference to Related Application

The present international application claims the priority based on Japanese Patent Application No. 2019-193830 filed with JPO on October 24, 2019, and the entire content of Japanese Patent Application No. 2019-193830 is incorporated into the present international application by reference herein.

### Technical Field

The present disclosure relates to a method for site-specifically modifying a specific region of Fc.

### Background Art

The inventors of the present disclosure have heretofore developed, as methods for site-specifically modifying IgG, a method using a IgG Fc region specifically binding peptide (IgBP) reagent (Patent Literature 1 and Non Patent Literature 1) or using a protein A-derived Z33 peptide reagent (CCAP method) (Patent Literature 2).

### Citation List

### Patent Literature

Patent Literature 1: International Publication No. WO 2016/186206
Patent Literature 2: International Publication No. WO 2018/230257

### Non Patent Literature

Non Patent Literature 1: Kishimoto, S., et al., Bioconjug. Chem. 30, 698-702 (2019).

### Summary of Invention

### Technical Problem

The CCAP method enables site specific binding of a peptide to a specific site of the Fc region, and has the advantage of not interfering with an antigen binding to an antigen binding region of an antibody. The inventors have produced an Fc-modified antibody by specifically binding a peptide to the Fc region utilizing the developed CCAP method, and advanced development for application of the antibody as a medicine and a diagnostic agent. As a result, the inventors have found that an antibody which is modified at a specific site of Fc by the CCAP method has the problem of being shortened in serum half-life as compared with an unmodified antibody. Further investigation of the cause of the problem has revealed that, while binding of IgBP to both the two binding sites, the Fc region has symmetry and usually includes two IgG affinity peptide (IgBP) binding sites, leads to a decrease in serum half-life, binding of IgBP to only one of the two binding sites eliminates the problem in the serum half-life. Accordingly, an objective of the present disclosure is to provide an antibody where only one of the two binding sites of the Fc region is selectively modified.

### Solution to Problem

In order to solve the problem, the inventors have tried a large number of experiment conditions, but selective modification of only one of two binding sites has been difficult to achieve by only improvements of reaction conditions of the CCAP method. The inventors have attempted purification and production by means of a carrier that bound to the Fc region. Specifically, the inventors have attempted purification of an Fc molecule where IgBP is bound to only one site (an Fc molecule not modified at one site) (monovalent Fc molecule), from IgBP-bound Fc molecules produced according to the CCAP method, by using a protein A binding column known to be bound to the Fc region. However, this attempt has not efficiently separated the antibodies depending on the valence of IgBP binding to the Fc region. Then, the inventors produced an IgBP-immobilized column and have attempted purification of the monovalent Fc molecule by allowing IgBP-bound Fc molecules produced according to the CCAP method to be adsorbed to the column. As a result, the monovalent Fc molecule was efficiently adsorbed to the column, while an Fc molecule (divalent Fc molecule) where IgBP is bound to both of two sites did not bind to the column and was eluted. The inventors have thus found a method that can efficiently obtain only the monovalent Fc molecule. Next, the inventors prepared a IgBP binding column to which only one site of Fc is bound, and confirmed whether or not the CCAP method can be performed on the IgBP binding column by applying a peptide reagent for CCAP to the column. As a result, it was revealed that modification by a peptide at only one binding site that was not bound to a carrier can be achieved by this method. From these findings, the inventors have succeeded in efficiently providing an Fc molecule where only one of two binding sites of Fc is selectively modified, and completed the invention of the present disclosure.

### Advantageous Effects of Invention

A method of the present disclosure can provide an Fc molecule where only one of two binding sites of Fc is selectively modified, at a high content, and thus can provide an Fc molecule having a long serum half-life.

### Brief Description of Drawings

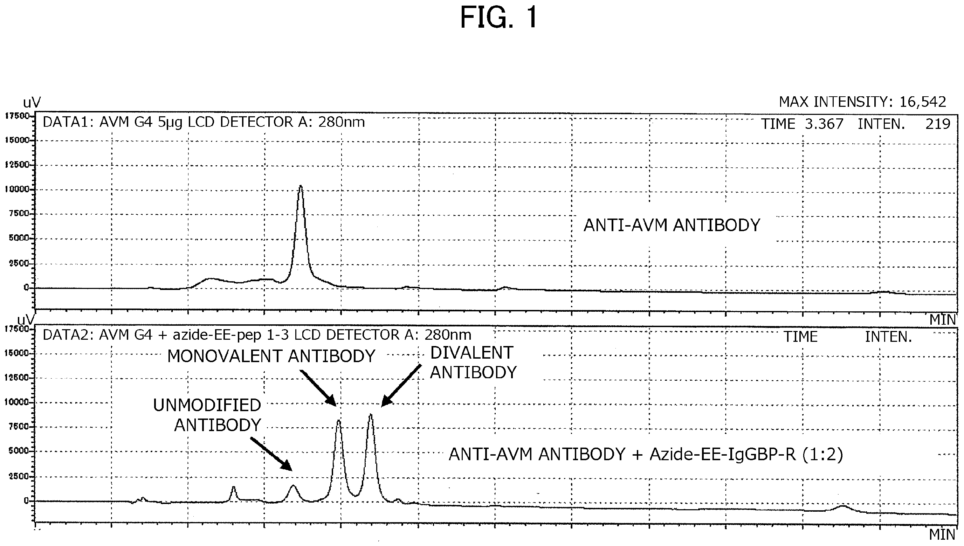

FIG. 1 shows graphs of the results of column chromatography obtained by injecting a portion (antibody 3 µg) of a solution obtained by reacting an anti-AVM (avermectin) antibody (human IgG4) with a CCAP reagent Azide-PEG4-EEGPDCAYH (succinimidyl glutaryl Lys) GELVWCTFH-NH₂ (Azide-EEIgBP), to an anion exchange column Shodex QA825 (Shodex), and eluted with-gradient NaCl (0 to 1 M). The top graph is the results of an unreacted anti-AVM antibody, and the bottom graph illustrates the results of an anti-AVM antibody reacted with Azide-EEIgBP. Respective arrows in the bottom graph represent peaks of the unmodified antibody, a monovalent antibody, and a divalent antibody that are eluted. The vertical axis represents the absorbance at 280 nm and the horizontal axis represents the elution time;

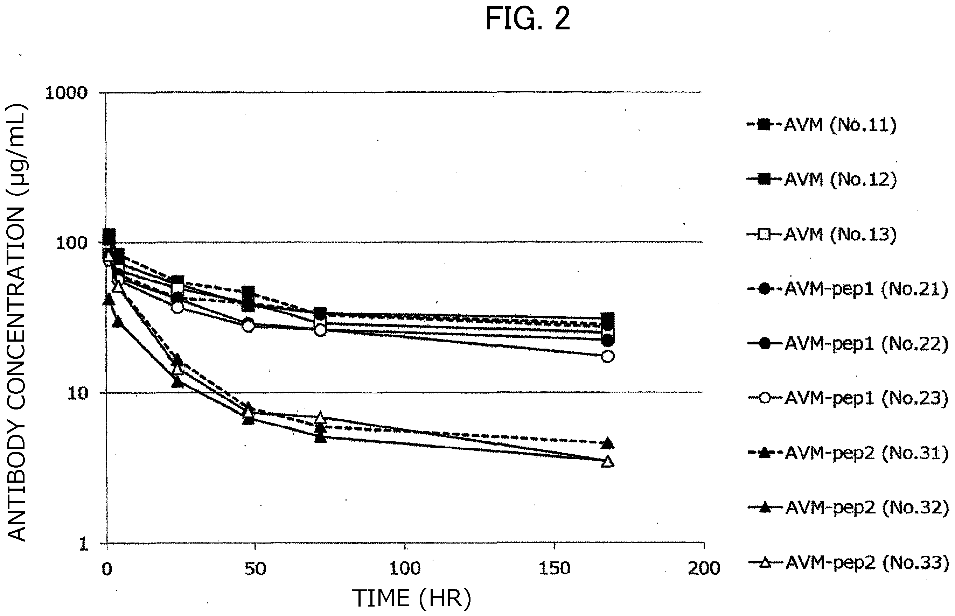

FIG. 2 shows the antibody concentrations in serums, analyzed 1 hour, 4 hours, 24 hours, 48 hours, 72 hours, and 168 hours after i.v. administration of a control antibody (AVM), a monovalent peptide antibody (AVM-pep1), and a divalent peptide antibody (AVM-pep2), in an amount of 5 mg/kg each, to ICR mice (three mice per group). The vertical axis represents the antibody concentration (µg/mL), and the horizontal axis represents the lapse time (hour) after administration. An AVM-administered group is represented by a square marker, an AVM-pep1-administered group is represented by a round marker, and an AVM-pep2-administered group is represented by a triangle marker;

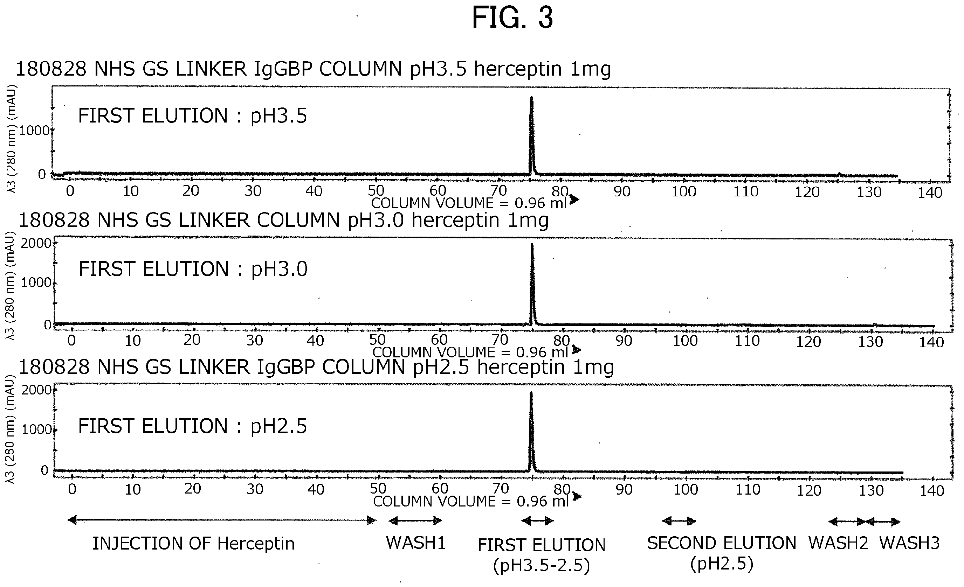

FIG. 3 shows chromatograms obtained by first elution at pH 3.5 (top graph), pH 3.0 (middle graph), and pH 2.5 (bottom graph) and a second acidic elution at pH 2.5, after injection of human IgG1 to an IgBP-immobilized column. The vertical axis represents the absorbance at 280 nm and the horizontal axis represents the lapse time. Operations performed in the column at the respective lapse times are shown at the lowermost portion of the graphs;

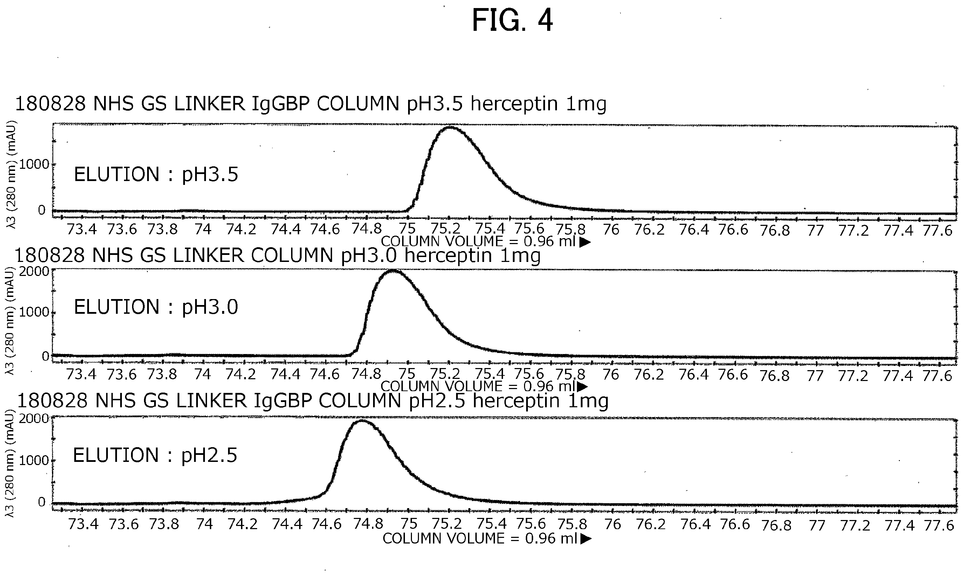

FIG. 4 shows enlarged chromatograms of the duration of the lapse time corresponding to "first elution" in FIG. 3. The chromatograms are the results of first elution at pH 3.5, pH 3.0, and pH 2.5 in the order from the top;

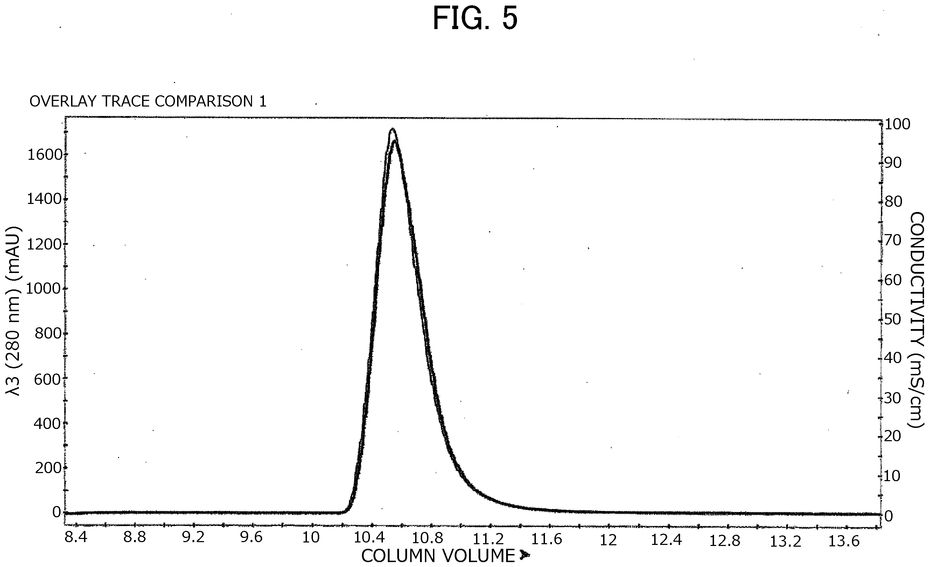

FIG. 5 shows a chromatogram eluted at pH 2.5 and pH 3.5 after injection of a human IgG1 antibody to a Z33-immobilized column. A gray line represents the result at pH 2.5 and a black line represents the result at pH 3.5 in the chromatogram. The vertical axis represents the absorbance at 280 nm and the horizontal axis represents the lapse time;

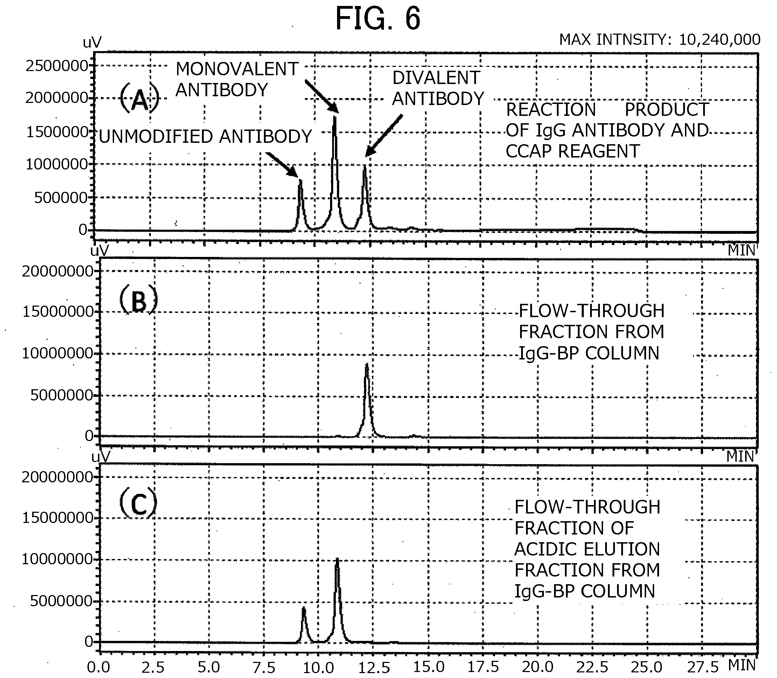

FIG. 6 shows graphs of the analysis results of hydrophobic chromatography of a reaction product of IgG and a CCAP reagent (IgBP-PEG7-N3-DBCO-MTZ-SG) which was fractioned by an IgBP column. (A) the reaction product of IgG (20 µM) and the CCAP reagent (60 µM) after 30 minutes reaction. (B) a flow-through fraction of the reaction product from the IgBP column. (C) an adsorption fraction to the IgBP column (acidic elution fraction). The vertical axis represents the absorbance at 280 nm and the horizontal axis represents the lapse time;

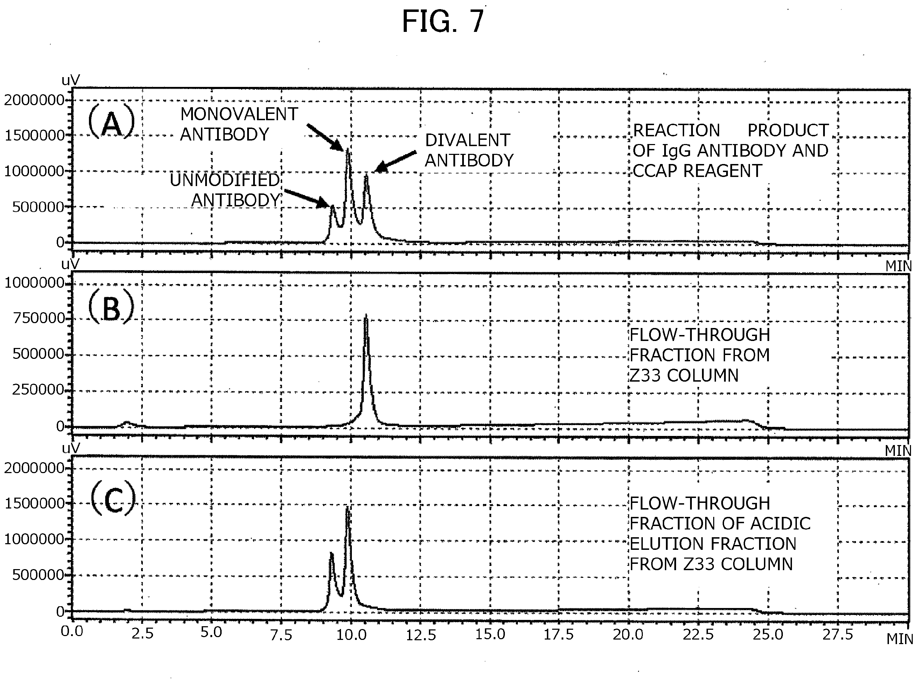

FIG. 7 shows graphs representing the analysis results of hydrophobic chromatography of a reaction product of IgG and a CCAP reagent (Z33-R7C-Mal-PEG4 MTZ-SG) which was fractioned by a Z33 column. (A) the reaction product of IgG (20 µM) and the CCAP reagent (60 µM) after 30 minutes reaction. (B) a flow-through fraction of the reaction product from the Z33 column. (C) an adsorption fraction to the Z33 column. The vertical axis represents the absorbance at 280 nm and the horizontal axis represents the lapse time;

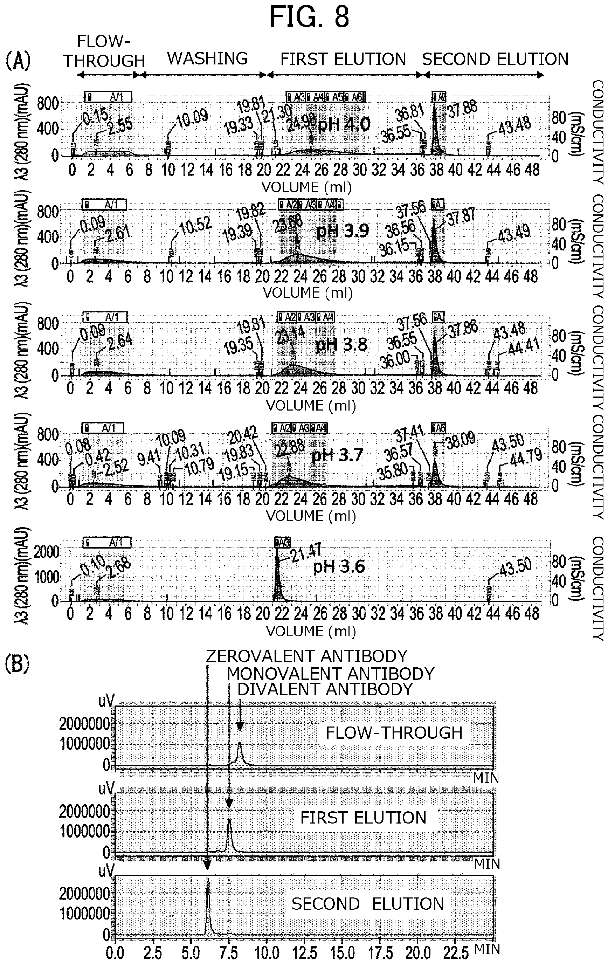

FIG. 8A and FIG. 8B are graphs illustrating purification of a monovalent antibody from a reaction product of Trastuzumab and a CCAP reagent by controlling an elution pH with using an IgBP column. The vertical axis represents the absorbance at 280 nm and the horizontal axis represents the lapse time. FIG. 8A illustrates the difference in elution pattern depending on pH of the first elution. The graphs are the results for first elution pH values of 4.0, 3.9, 3.8, 3.7, and 3.6 in the order from the top to the bottom. FIG. 8B shows graphs of the content of zerovalent (unmodified), monovalent, and divalent antibodies analyzed by cation exchange chromatography in a flow-through fraction from the first elution at pH 3.8 (top graph), a fraction by the first elution (pH 3.8) (middle graph), and a fraction by the second elution (pH 3.5) (bottom graph);

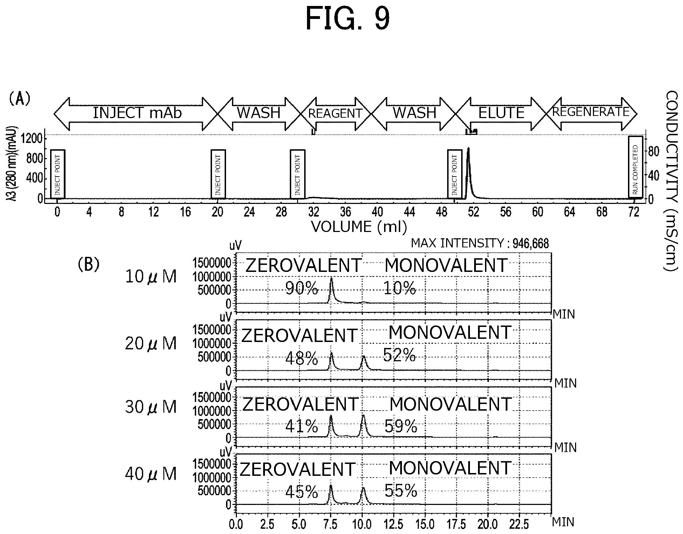

FIG. 9A and FIG. 9B are graphs illustrating the results of an automotive CCAP reaction on an IgBP column connected to a high-performance liquid chromatograph (NGC, BioRad). FIG. 9A illustrates a flow chart of an automation process of modification of an antibody by an automotive CCAP reaction. FIG. 9B shows graphs of the analysis results of cation exchange chromatography of the eluted fractions which differ depending on concentrations of a CCAP reagent in the automotive CCAP reaction. The graphs are the results for the concentrations of the CCAP reagent of 10 µM, 20 µM, 30 µM, and 40 µM in the order from the top to the bottom. The vertical axis represents the absorbance at 280 nm and the horizontal axis represents the lapse time. The numerical values in each of the graphs respectively represent the valence of IgBP in the resulting antibody and their proportions in yield;

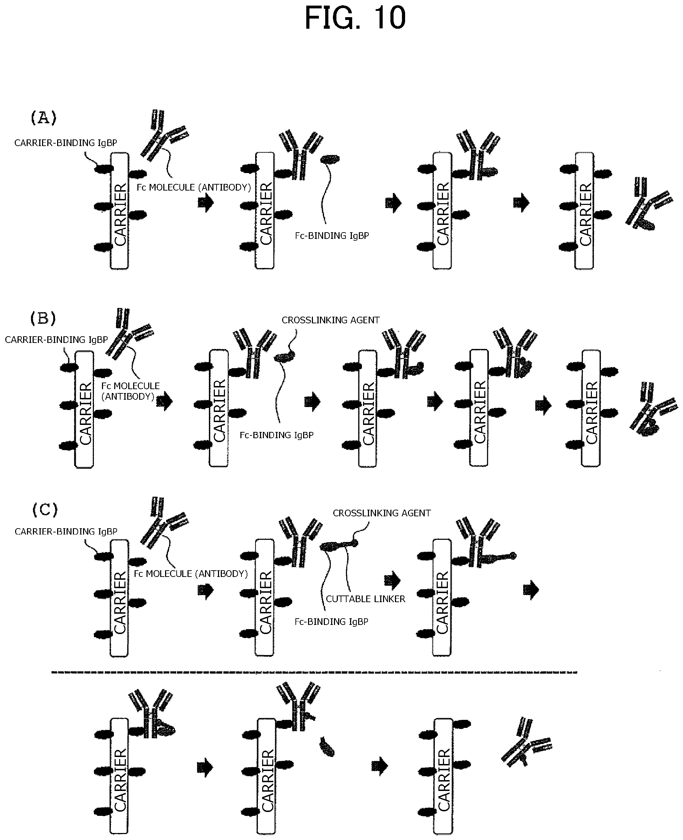

FIG. 10A is a schematic diagram of a method for producing an IgBP-bound Fc molecule of the present disclosure on a carrier in the case of using an antibody as an Fc molecule. First, an Fc molecule is contacted with a carrier to which Carrier-binding IgBP is bound, which lead the carrier to be bound to the Fc molecule via Carrier-binding IgBP. Then, Fc-binding IgBP is contacted therewith so that the Fc-binding IgBP can bind to the site of Fc chain to which no IgBP is bound. Finally, a bond between the Carrier-binding IgBP and the Fc molecule is cut, and the Fc molecule to which one Antibody-binding IgBP per molecule is bound, is recovered;
FIG. 10B is a schematic diagram of a method for producing an IgBP-bound Fc molecule with a CCAP reagent, in the method of FIG. 10A of the top diagram. In addition to the step in FIG. 10A, a step of binding Fc-binding IgBP to an Fc molecule, and then covalently binding a crosslinking agent to the Fc molecule is included;
FIG. 10C is a schematic diagram of a method for producing a monovalent functional group-bound Fc molecule by using a reagent in which Antibody-binding IgBP and a crosslinking agent are linked via a cleavable linker. Fc-binding IgBP is bound to an Fc molecule, and then a crosslinking agent and the Fc molecule are covalently bound. Since the crosslinking agent and Antibody-binding IgBP are linked via a cleavable linker, the Fc-binding IgBP is dissociated from the Fc molecule by cleaving the linker moiety, which enables to introduce a monovalent functional group into the Fc molecule without including IgBP; and

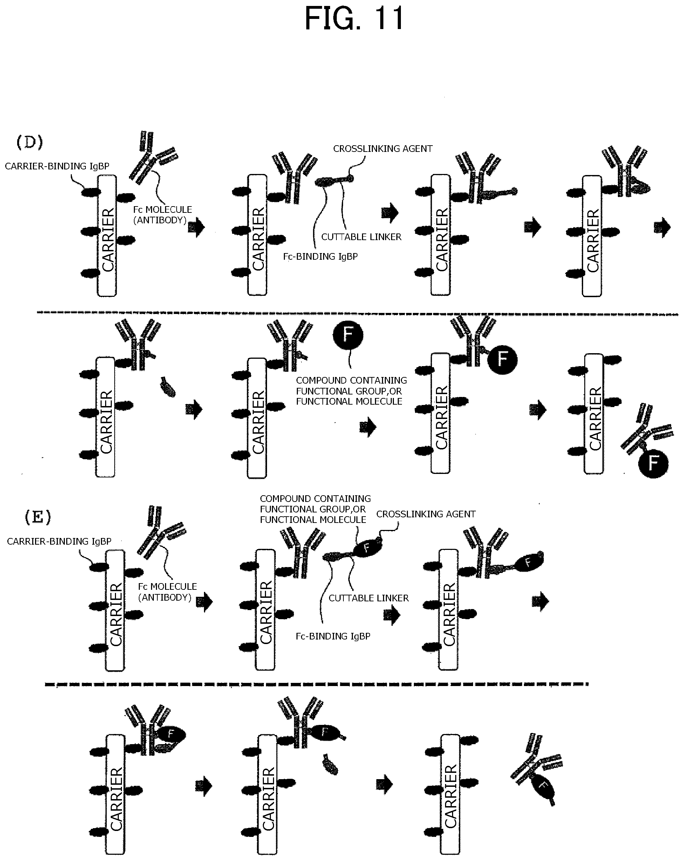

FIG. 11D is a schematic diagram of a method for producing an Fc molecule to which a monovalent functional group or a monovalent functional molecule is bound. This method includes a step of binding a functional group-containing compound or a functional molecule to the Fc molecule after cleaving the linker moiety in FIG. 10C. In this diagram, a functional group or a functional molecule represented by "F" is bound to the Fc molecule, which is then recovered from a carrier, but the functional group or the functional molecule may be bound to the Fc molecule after recovery from the carrier; and
FIG. 11E is a schematic diagram of a method for producing an Fc molecule to which a monovalent functional group or a functional molecule is bound by using a regent in which Fc-binding IgBP and a functional group-containing group or a functional molecule are bound via a cleavable linker and the functional group-containing group or the functional molecule is further bound to a crosslinking agent. As in FIG. 10C, the linker can be cleaved to dissociateAntibody-binding IgBP after covalent binding of the crosslinking agent to the Fc molecule, thereby enable to obtain a monovalent functional group-bound Fc molecule or a functional molecule-bound Fc molecule without IgBP bound.

### Description of Embodiments

The present disclosure is based on the finding where IgBP (Carrier-binding IgBP) on a carrier can be bound to a binding site of one Fc in two Fc chains constituting an Fc molecule to thereby efficiently separate an antibody to which two IgBPs (IgBPs for Fc molecule binding) to be introduced into the Fc chains are bound and an antibody to which one or zero of such IgBPs are bound, and the finding where Fc-binding IgBP can be bound to an Fc chain bound to Carrier-binding IgBP to thereby bind only one IgBP molecule to an Fc molecule. In other words, the present disclosure provides a method for producing an Fc molecule to which only one IgBP molecule is bound, by use of a carrier to which Carrier-binding IgBP is bound.

The "Fc region of IgG" and "Fc region" herein have the same meanings, and each typically mean a fragment located at the C terminal, the fragment being obtained as a product obtained by treating IgG with a proteolytic enzyme papain. The Fc region generally includes two symmetric Fc chains, and two Fc chains for forming the Fc region does not need to be strictly identical. The "Fc chain" means a monomer of a dimer for forming a fragment located at the C terminal, the fragment being obtained as a product obtained by treating IgG with a proteolytic enzyme papain. The Fc region of IgG herein does not need to be a wild type full-length Fc region of IgG, and may correspond to a shortened form or a variant form, or a fused product with other substance, as long as the region retains a binding force to an IgG affinity peptide to be specifically bound to an Fc region of IgG used. The method described herein may include binding a crosslinking agent-bound IgG affinity peptide and an Fc region of IgG by use of affinity and then forming a covalent bond of the IgG affinity peptide and the Fc region of IgG by a crosslinking agent (CCAP method). Therefore, the "binding force to an IgG affinity peptide to be specifically bound to an Fc region of IgG used" means both an ability of the IgG affinity peptide and the Fc region of IgG to be bound by affinity and an ability of the IgG affinity peptide and the Fc region of IgG to be covalently bound. For example, the "Fc region of IgG" herein refers to a site that includes some (1 to 10, 1 to 5, or 1 to 3) amino acids, except for any position to which an IgG affinity peptide used is to be bound, in an Fc region of wild type IgG, and that does not have any influence on the affinity, in which an amino acid at a site other than an amino acid (usually Lys) involving a covalent bond may be, if necessary, substituted, added or inserted, or deleted.

The "molecule having an Fc region of IgG" and "Fc molecule" herein have the same meanings, and each mean a peptide, a protein, or other composite, including the Fc region of IgG, and encompass, not only wild type or artificial IgG and a variant of such IgG, but also a fused product of an Fc region of IgG typified by an Fc fusion protein and other substance (active ingredient, drug, protein, low molecular compound, medium molecular compound, high molecular compound, matrix, lipid, liposome, nanoparticle, vehicle for DDS, nucleic acid and/or peptide), and a molecule containing only an Fc region. For example, in a case where the Fc molecule is an Fc fusion protein, examples of the protein or peptide to be fused with Fc include a receptor, cytokine, interleukin, blood-coagulation factor VIII, CTLA4, human lactoferrin, a TNF receptor, or LFA-3, or a portion thereof (preferably a target-binding moiety). The "Fc molecule composition" herein means a composition containing a plurality of Fc molecules.

The "IgG" herein may be IgG of mammals, for example, primates such as humans and chimpanzees, laboratory animals such as rats, mice, and rabbits, livestock animals such as pigs, cows, horses, sheep, and goats, and pet animals such as dogs and cats, and is preferably human IgG (IgG1, IgG2, IgG3 or IgG4). The IgG herein is preferably human IgG1, IgG2, or IgG4, or rabbit IgG, and particularly preferably human IgG1, IgG2, or IgG4.

The "IgG affinity peptide to be specifically bound to an Fc region of IgG" and "IgBP" herein have the same meanings, and each mean a peptide to be specifically bound to an Fc region of IgG. IgBP is preferably a peptide to be bound to a site selected from Lys248, Lys246, Lys338, Lys288, Lys290, Lys360, Lys414, and Lys439 following Eu numbering of Fc and/or an adjacent region of the site, preferably Lys248 and/or an adjacent region thereof, or a binding region of protein A. For example, IgBP may be a partial peptide of protein A having Fc binding ability, or a variant of the peptide. Specific examples of such a peptide are described in International Publication Nos. WO 2008/054030, WO 2013/027796, WO 2016/186206, and WO 2018/230257, and Kyohei Muguruma and others, ACS Omega (2019);4 (11):14390-14397, and can be appropriately prepared according to any method described in these Literatures.

The method described herein is to utilize a carrier to which IgBP (Carrier-binding IgBP) is bound, thereby efficiently binding IgBP (Fc-binding IgBP) that is the same as or different from the IgBP, to only one site of two binding sites of an Fc molecule. Hereinafter, IgBP to be bound to a carrier refers to "Carrier-binding IgBP". IgBP to be bound to an Fc molecule is referred to as "Fc-binding IgBP". The carrier to which Carrier-binding IgBP is bound is referred to as "IgBP-bound carrier", and the Fc molecule to which Fc-binding IgBP is bound is referred to as "IgBP-bound Fc molecule".

Specific examples of Carrier-binding IgBP can include the following (i) or (ii) peptide to be specifically bound to an Fc region of IgG. X^{m} (m is an integer) herein represents an amino acid. "X^{m}ₙ" represents binding of n amino acids X^{m}(n), and "X^{m}" without n designated represents one amino acid X^{m}. When n is 2 or more, a plurality of X^{m}(s) may be each independently the same or different amino acid. When n is "p to q", the presence of p to q amino acids X^{m}(p to q) is shown. (i) A peptide represented by the following formula (I):

NH₂-(Linker)-(X¹₁₋₃)-C-(X²)-(X³)-(X⁴)-(X⁵)-G-(X⁶)-L-(X⁷)-W-C-(X⁸₁₋₃) ... (I)

in the formula (I), (Linker) represents a linker, one to three X¹(S), X², X³, X⁴, X⁵, X⁶, X⁷, and one to three X⁸(s) each mutually independently represent the same or different amino acid residue,
each X¹, X², X³, and each X⁸ mutually independently represent any amino acid residues that are the same or different and are other than C,
X⁴ is H, R, S, or T,
X⁵ is one amino acid residue selected from K, C, D, E, R, V, F, L, 2-aminosuberic acid, Dpr, Orn, AcOrn, AcDab, Dab, Nle, Nva, Tie, Ala(t-Bu), and Cha,
X⁶ is E, N, R, or D, and
X⁷ is I or V.

In the formula (I), Linker is (GSGGS)₁₋₃, (SGSGS)₁₋₃, (GGGGS)₁₋₃, or (PEG)₂₋₁₀ (preferably (PEG)₄), or absent. A (-C(=O)NH₂) group may be formed by binding an amino group to a carboxyl terminal (-COOH) at the C terminal of the formula (I) or Linker (defined in the same manner as described above) may be arbitrarily inserted between the carboxyl terminal and an amino group, for the purpose of binding with a carrier. When Linker is present at the C terminal, no Linker at the N terminal side may be present. In other words, (X¹₁₋₃)-C-(X²)-(X³)-(X⁴)-(X⁵)-G-(X⁶)-L-(X⁷)-W-C-(X⁸₁₋₃)-(Linker)-NH₂ may also be formed. In the formula (I), the amino group at the N terminal may be acetylated (in the case, a Lys residue is introduced at a proper position in the vicinity of the N terminal in Linker at the N terminal side).

Examples of the peptide represented by the formula (I) preferably include the following peptides.
[1] X¹₁₋₃ is an amino acid sequence represented by (S, G, F, or none)-(D, G, A, S, P, Hcy, or none)-(S, D, T, N, E, or R).
[2] X¹₁₋₃ is D, GPD, R, GPR, SPD, GDD, GPS, SDD, RGN, G-Hcy-D, RGP, or GPD.
[3] X¹₁₋₃ is D or GPD.
[4] X² is A, S, or T.
[5] X² is A or T.
[6] X² is A.
[7] X³ is Y or W.
[8] X³ is Y.
[9] X⁴ is H.
[10] X⁵ is one amino acid residue selected from A, R, K, C, D, E, L, 2-aminosuberic acid, Dpr, R, F, 2-aminosuberic acid, Dpr, AcOrn, AcDab, Dab, Nle, Nva, Ala(t-Bu), and Cha.
[11] X⁵ is K, R, AcOrn.
[12] X⁵ is one amino acid residue selected from V, Dab, F, R, L, Nva, Nle, Ala(t-Bu), and Cha.
[13] X⁵ is one amino acid residue selected from F, R, L, Nva, Nle, Ala(t-Bu), and Cha.
[14] X⁵ is one amino acid residue selected from L, Ala(t-Bu), and Cha.
[15] X⁶ is E or N.
[16] X⁶ is E.
[17] X⁷ is V.
[18] X⁸₁₋₃ is (S, T, or D)-(H, G, Y, T, N, D, F, Hcy, or none)-(Y, F, H, M, or none).
[19] X⁸₁₋₃ is T, TFH, S, SFH, THH, TFY, TYH, or T-Hcy-H.
[20] X⁸₁₋₃ is T or TFH.

The peptide represented by the formula (I) may be any one of the above conditions, or any combination of two or more of the above conditions, and may be, for example, a peptide satisfying any condition described below: [8] and [9]; [8] and [17]; [9] and [17]; [8] and [9] and [17]; or a combination of such any condition and any one of [10] to [14]. More specific examples of the peptide can include the following peptides (hereinafter, X⁵ is the same as described above; and each peptide may have a NH₂-(Linker)- group at the N terminal and may have a -NH₂ group or NH₂-(Linker)- group at the C terminal):
1) DCAYHX⁵GELVWCT (SEQ ID No. 1)
2) GPDCAYHX⁵GELVWCTFH (SEQ ID No. 2)
3) RCAYHX⁵GELVWCS (SEQ ID No. 3)
4) GPRCAYHX⁵GELVWCSFH (SEQ ID No. 4)
5) SPDCAYHX⁵GELVWCTFH (SEQ ID No. 5)
6) GDDCAYHX⁵GELVWCTFH (SEQ ID No. 6)
7) GPSCAYHX⁵GELVWCTFH (SEQ ID No. 7)
8) GPDCAYHX⁵GELVWCSFH (SEQ ID No. 8)
9) GPDCAYHX⁵GELVWCTHH (SEQ ID No. 9)
10) GPDCAYHX⁵GELVWCTFY (SEQ ID No. 10)
11) SPDCAYHX⁵GELVWCTFY (SEQ ID No. 11)
12) SDDCAYHX⁵GELVWCTFY (SEQ ID No. 12)
13) RGNCAYHX⁵GQLVWCTYH (SEQ ID No. 13)
14) G-Hcy-DCAYHX⁵GELVWCT-Hcy-H (SEQ ID No. 14)
15) RRGPDCAYHX⁵GELVWCTFH (SEQ ID No. 15)
16) DCTYHX⁵GNLVWCT (SEQ ID No. 16)
17) DCAYHX⁵GNLVWCT (SEQ ID No. 17)
18) DCTYHX⁵GELVWCT (SEQ ID No. 18)
19) DCAWHX⁵GELVWCT (SEQ ID No. 19)
20) DCTYTX⁵GNLVWCT (SEQ ID No. 20),
21) DCAYTX⁵GNLVWCT (SEQ ID No. 21),
22) DCSYTX⁵GNLVWCT (SEQ ID No. 22),
23) DCTWTX⁵GNLVWCT (SEQ ID No. 23),
24) DCTYHX⁵GNLVWCT (SEQ ID No. 24),
25) DCTYRX⁵GNLVWCT (SEQ ID No. 25),
26) DCTYSX⁵GNLVWCT (SEQ ID No. 26),
27) DCTYTX⁵GNLVWCT (SEQ ID No. 27),
28) DCTYTX⁵GELVWCT (SEQ ID No. 28),
29) DCTYTX⁵GRLVWCT (SEQ ID No. 29),
30) DCTYTX⁵GDLVWCT (SEQ ID No. 30),
31) DCTYTX⁵GNLIWCT (SEQ ID No. 31),
32) DCAYHRGELVWCT (SEQ ID No. 32)
33) GPDCAYHRGELVWCTFH (SEQ ID No. 33)
34) RCAYHRGELVWCS (SEQ ID No. 34)
35) GPRCAYHRGELVWCSFH (SEQ ID No. 35)
36) SPDCAYHRGELVWCTFH (SEQ ID No. 36)
37) GDDCAYHRGELVWCTFH (SEQ ID No. 37)
38) GPSCAYHRGELVWCTFH (SEQ ID No. 38)
39) GPDCAYHRGELVWCSFH (SEQ ID No. 39)
40) GPDCAYHRGELVWCTHH (SEQ ID No. 40)
41) GPDCAYHRGELVWCTFY (SEQ ID No. 41)
42) SPDCAYHRGELVWCTFY (SEQ ID No. 42)
43) SDDCAYHRGELVWCTFY (SEQ ID No. 43)
44) DCTYHRGNLVWCT (SEQ ID No. 44)
45) DCAYHRGNLVWCT (SEQ ID No. 45)
46) DCTYHRGELVWCT (SEQ ID No. 46)
47) DCAWHRGELVWCT (SEQ ID No. 47)
48) DCTYTNGNLVWCT (SEQ ID No. 48)
49) DCAYTNGNLVWCT (SEQ ID No. 49)
50) DCSYTNGNLVWCT (SEQ ID No. 50)
51) DCTWTNGNLVWCT (SEQ ID No. 51)
52) DCTYHNGNLVWCT (SEQ ID No. 52)
53) DCTYRNGNLVWCT (SEQ ID No. 53)
54) DCTYSNGNLVWCT (SEQ ID No. 54)
55) DCTYTRGNLVWCT (SEQ ID No. 55)
56) DCTYTNGELVWCT (SEQ ID No. 56)
57) DCTYTNGRLVWCT (SEQ ID No. 57)
58) DCTYTNGDLVWCT (SEQ ID No. 58)
59) DCTYTNGNLIWCT (SEQ ID No. 59)

In one example, a peptide having the following structure can be used as Carrier-binding IgBP.
GSGGS-GPDCAYHRGELVWCTFH-NH₂
(PEG)₄-GPDCAYHRGELVWCTFH-NH₂
GSGGS-DCAYHRGELVWCT-NH₂
(PEG)₄-DCAYHRGELVWCT-NH₂

(ii) A peptide represented by the following formula (II), or a peptide containing an amino acid sequence of (II), where one or several amino acids are each added, deleted, and/or substituted at a position other than X⁹ to X¹³:

X⁹₁₋₂NMQX¹⁰QRRFYEALHDPNLNEEQRNAX¹¹IX¹²SIRDDX¹³-(Linker 2)-CONH₂ (SEQ ID No. 60) ... (II)

in the formula (II), (Linker 2) represents a linker,
X⁹₁₋₂ is selected from the group consisting of GF, AF, VF, LF, IF, MF, PF, FF, WF, KF, Orn-F, CF, DF, EF, beta-alanine-F, 2-aminosuberic acid-F, Dpr-F, NH₂-(PEG)ₙ-CO(wherein n = 1-50)-F, F, K, Orn, C, D, E, 2-aminosuberic acid, Dpr, and Acetyl-K,
X¹⁰ is C or Q,
X¹¹ and X¹² are each independently selected from the group consisting of R, H, D, E, S, T, N, Q, Y, and C, and
X¹³ is C or P, or absent.

In the formula (II), Linker 2 is (GSGGS)₁₋₃, (SGSGS)₁₋₃, (GGGGS)₁₋₃, or (PEG)₂₋₁₀-Lys (preferably (PEG)₄-Lys), or absent. The terminal (-NH²) of the N-terminal amino acid of the formula (II) may be acetylated to form a (CH₃-C(=O)-NH-) group. Linker 2 may be bound to the amino terminal (in the case, a Lys residue is introduced at a proper position in the vicinity of the N terminal in Linker at the N terminal side), and Linker 2 at the C terminal is optionally present.

Examples of the peptide having the amino acid sequence represented by the formula (II) preferably include the following peptides.
[21] X⁹ is selected from the group consisting of GF, AF, βAlaF, NH₂-(PEG)ₙ-CO(n = 2 to 10)-F, F, K, Orn, C, and Dpr.
[22] X⁹ is selected from the group consisting of GF, F, and K.
[23] X¹⁰ is Q.
[24] X¹¹ and X¹² are each independently selected from the group consisting of R, H, and E.
[25] X¹¹ and X¹² are each R.

More specific examples of the peptide having the amino acid sequence represented by the formula (II) can include the following peptides:
60) FNMQCQRRFYEALHDPNLNEEQRNARIRSIRDDC (SEQ ID No. 62),
61) GFNMQCQRRFYEALHDPNLNEEQRNARIRSIRDDC (SEQ ID No. 63),
62) KNMQCQRRFYEALHDPNLNEEQRNARIRSIRDDC (SEQ ID No. 64),
63) GFNMQCQKRFYEALHDPNLNEEQRNARIRSIRDDC (SEQ ID No. 65),
64) KNMQCQKRFYEALHDPNLNEEQRNARIRSIRDDC (SEQ ID No. 66),
65) FNMQQQRRFYEALHDPNLNEEQRNARIRSIRDD (SEQ ID No. 67), or
66) GKNMQCQRRFYEALHDPNLNEEQRNARIRSIRDDC (SEQ ID No. 68).

For example, a peptide having the following structure can be used as Carrier-binding IgBP.
Acetyl-FNMQQQRRFYEALHDPNLNEEQRNARIRSIRDDP-SGSGSK-NH₂ Acetyl-FNMQCQRRFYEALHDPNLNEEQRNARIRSIRDDC-SGSGSK-NH₂
Acetyl-FNMQQQRRFYEALHDPNLNEEQRNARIRSIRDDP-(PEG)₄-Lys-NH₂ Acetyl-FNMQCQRRFYEALHDPNLNEEQRNARIRSIRDDC-(PEG)₄-Lys-NH₂

Carrier-binding IgBP has at least one amino group (-NH₂), for the purpose of covalent binding with a carrier. Such an amino group is preferably the amino group at the N terminal, and may be a side chain amino group of a lysine residue, a cysteine residue, an aspartic acid residue, a glutamic acid residue, a 2-aminosuberic acid, Dpr, or an arginine residue (for example, located in the linker) in the vicinity of the N terminal or C terminal, as long as binding with a carrier can be formed.

Specific examples of Fc-binding IgBP can include the following peptide (iii) or (iv) to be specifically bound to an Fc region of IgG.

(iii) A peptide represented by the following formula (I'):

Z-[(Linker 3)-(X¹₁₋₃)-C-(X²)-(X³)-(X⁴)-(X⁵)-G-(X⁶)-L-(X⁷)-W-C-(X⁸₁₋₃)] ... (I')

in the formula (I'), Z represents a functional group and is bound to any moiety of a structure represented by [(Linker 3)-(X¹₁₋₃)-C-(X²)-(X³)-(X⁴)-(X⁵)-G-(X⁶)-L-(X⁷)-W-C-(X⁸₁₋₃)], (Linker 3) represents a linker, one to three X¹(S), X², X³, X⁴, X⁵, X⁶, X⁷, and one to three X⁸(s) each mutually independently represent the same or different amino acid residue,
each X¹, X², X³, and each X⁸ mutually independently represent any amino acid residues that are the same or different and are other than C,
X⁴ is H, R, S, or T,
X⁵ is one amino acid residue selected from K, C, D, E, R, V, F, L, 2-aminosuberic acid, Dpr, Orn, AcOrn, AcDab, Dab, Nle, Nva, Tie, Ala(t-Bu), and Cha,
X⁶ is E, N, R, or D, and
X⁷ is I or V.

The peptide represented by the formula (I') may have a functional group at the C terminal, instead of having a functional group at the N terminal. In other words, the peptide represented by the formula (I') may be a peptide represented by the following formula (I").

[(X¹₁₋₃)-C-(X²)-(X³)-(X⁴)-(X⁵)-G-(X⁶)-L-(X⁷)-W-C-(X⁸₁₋₃)-(Linker 3)]-Z ... (I")

In the formula (I"), Z represents a functional group and is bound to any moiety of a structure represented by [(X¹₁₋₃)-C-(X²)-(X³)-(X⁴)-(X⁵)-G-(X⁶)-L-(X⁷)-W-C-(X⁸₁-₃)-(Linker 3)], (Linker 3) represents a linker, one to three X¹(S), X², X³, X⁴, X⁵, X⁶, X⁷, and one to three X⁸(s) each mutually independently represent the same or different amino acid residue,
each X¹, X², X³, and each X⁸ mutually independently represent any amino acid residues that are the same or different and are other than C,
X⁴ is H, R, S, or T,
X⁵ is one amino acid residue selected from K, C, D, E, R, V, F, L, 2-aminosuberic acid, Dpr, Orn, AcOrn, AcDab, Dab, Nle, Nva, Tie, Ala(t-Bu), and Cha,
X⁶ is E, N, R, or D, and
X⁷ is I or V.

In the formula (I') and the formula (I"), Linker 3 is RRRGS, EEGGS or (PEG)₁₋₈ (preferably (PEG)₄), or absent. An amino group may be bound to the terminal (-COOH) of the C-terminal amino acid of the formula (I') to form a (-C(=O)NH₂) group. An acetyl group may be bound to the terminal (-NH²) of the N-terminal amino acid of the formula (I") to form a (CH₃-C(=O)-NH-) group.

The respective peptides having the amino acid sequences represented by the formula (I') and the formula (I") may be each Z-(Linker 3)-(X¹₁₋₃)-C-(X²)-(X³)-(X⁴)-(X⁵)-G-(X⁶)-L-(X⁷)-W-C-(X⁸₁₋₃) or (X¹₁₋₃)-C-(X²)-(X³)-(X⁴)-(X⁵)-G-(X⁶)-L-(X⁷)-W-C-(X⁸₁₋₃)-(Linker 3)-Z. A preferable amino acid sequence is the same as the preferable amino acid sequence in the peptide represented by the formula (I).

Examples of Fc-binding IgBP can include the following peptide. Acetyl-K(Z)-RRRGS-GPDCAYHKGELVWCTFH-NH₂ Acetyl-K(Z)-EEGGS-GPDCAYHKGELVWCTFH-NH₂ Acetyl-K(Z)-(PEG)₄-GPDCAYHKGELVWCTFH-NH₂ Maleimide-RRRGS-GPDCAYHKGELVWCTFH-NH₂ Maleimide-EEGGS-GPDCAYHKGELVWCTFH-NH₂ Maleimide-(PEG)₄-GPDCAYHKGELVWCTFH-NH₂ DBCO-RRRGS-GPDCAYHKGELVWCTFH-NH₂ DBCO-EEGGS-GPDCAYHKGELVWCTFH-NH₂ DBCO-(PEG)₄-GPDCAYHKGELVWCTFH-NH₂ Tetrazine-RRRGS-GPDCAYHKGELVWCTFH-NH₂ Tetrazine-EEGGS-GPDCAYHKGELVWCTFH-NH₂ Tetrazine-(PEG)₄-GPDCAYHKGELVWCTFH-NH₂ TCO-RRRGS-GPDCAYHKGELVWCTFH-NH₂ TCO-EEGGS-GPDCAYHKGELVWCTFH-NH₂ TCO-(PEG)₄-GPDCAYHKGELVWCTFH-NH₂ Acetyl-K(Z)RRRGS-DCAYHKGELVWCT-NH₂ Acetyl-K(Z)EEGGS-DCAYHKGELVWCT-NH₂ Acetyl-K(Z)-(PEG)₄-DCAYHKGELVWCT-NH₂ Maleimide-RRRGS-DCAYHKGELVWCT-NH₂ Maleimide-EEGGS-DCAYHKGELVWCT-NH₂ Maleimide-(PEG)₄-DCAYHKGELVWCT-NH₂ DBCO-RRRGS-DCAYHKGELVWCT-NH₂ DBCO-EEGGS-DCAYHKGELVWCT-NH₂ DBCO-(PEG)₄-DCAYHKGELVWCT-NH₂ Tetrazine-RRRGS-DCAYHKGELVWCT-NH₂ Tetrazine-EEGGS-DCAYHKGELVWCT-NH₂ Tetrazine-(PEG)₄-DCAYHKGELVWCT-NH₂ TCO-RRRGS-DCAYHKGELVWCT-NH₂ TCO-EEGGS-DCAYHKGELVWCT-NH₂ TCO-(PEG)₄-DCAYHKGELVWCT-NH₂

(iv) A peptide containing an amino acid sequence represented by the following formula (II'), or a peptide containing an amino acid sequence of (II'), where one or several amino acids are each added, deleted, and/or substituted at a position other than X⁹ to X¹⁴:

X⁹₁₋₂NMQX¹⁰QX¹⁴RFYEALHDPNLNEEQRNAX¹¹IX¹²SIRDDX¹³-(Linker 2)-NH₂ (SEQ ID No. 61) ... (II')

in the formula (II'), (Linker 2) represents a linker,
X⁹₁₋₂ is selected from the group consisting of GF, AF, VF, LF, IF, MF, PF, FF, WF, KF, Orn-F, CF, DF, EF, beta-alanine-F, 2-aminosuberic acid-F, Dpr-F, NH₂-(PEG)ₙ-CO(wherein n = 1-50)-F, F, K, Orn, C, D, E, 2-aminosuberic acid, Dpr, and Acetyl-K,
X¹⁰ is C or Q,
X¹¹ and X¹² are each independently selected from the group consisting of R, H, D, E, S, T, N, Q, Y, C, and K(Z),
X¹³ is C or P, or absent,
X¹⁴ is R, C, K, or K(Z), and
Z is a functional group.

In the formula (II'), Linker 2 is SGSGSK, SRRCR, SRRK(Z)R, SRRCRRCRRC, SRRK(Z)RRK(Z)RRK(Z), or (PEG)₁₋₈-Lys (preferably (PEG)₄-Lys), or absent. The terminal (-NH²) of the N-terminal amino acid of the formula (II') may be acetylated to form a (CH₃-C(=O)-NH-) group. The Cys residue (C) contained in the linker may be, if necessary, bound to other functional molecule via a maleimide group.

Examples of the peptide having the amino acid sequence represented by the formula (II') preferably include the following peptides.
[a] X⁹ is selected from the group consisting of GF, AF, βAlaF, NH₂-(PEG)ₙ-CO(n=1 to 50)-F, F, K, Orn, C, Dpr, and Acetyl-K.
[b] X⁹ is selected from the group consisting of GF, F, and Acetyl-K.
[c] X¹⁰ is Q.
[d] X¹¹ and X¹² are each independently selected from the group consisting of R, H, and E.
[e] X¹¹ is R.
[f] X¹² is R or K(Z) (preferably Z is azide).

More specific examples of the peptide having the amino acid sequence represented by the formula (II') can include the peptides described in 60) to 66) (provided that the lysine residue contained may be, if necessary, bound to a functional group). Specific examples of Fc-binding IgBP can include the following peptides: FNMQQQCRFYEALHDPNLNEEQRNARIRSIRDD-NH₂ FNMQQQRRFYEALHDPNLNEEQRNARIRSIRDDC-NH₂ FNMQQQRRFYEALHDPNLNEEQRNARIRSIRDDP-SRRK(Z)R-NH₂ FNMQQQRRFYEALHDPNLNEEQRNARIRSIRDDP-SRRCR-NH₂ FNMQQQCRFYEALHDPNLNEEQRNARICSIRDDP-SRRCRRCRRC-NH₂ FNMQQQK(Z)RFYEALHDPNLNEEQRNARIK(Z)SIRDDP-SRRK(Z)RRK(Z)RRK(Z)-NH₂ Acetyl-KNMQQQCRFYEALHDPNLNEEQRNARIRSIRDD-NH₂ Acetyl-KNMQQQRRFYEALHDPNLNEEQRNARIRSIRDDC-NH₂ Acetyl-KNMQQQRRFYEALHDPNLNEEQRNARIRSIRDDP-SRRK(Z)R-NH₂ Acetyl-KNMQQQRRFYEALHDPNLNEEQRNARIRSIRDDP-SRRCR-NH₂ Acetyl-KNMQQQCRFYEALHDPNLNEEQRNARICSIRDDP-SRRCRRCRRC-NH₂ Acetyl-KNMQQQK(Z)RFYEALHDPNLNEEQRNARIK(Z)SIRDDP-SRRK(Z)RRK(Z)RRK(Z)-NH₂ GFNMQQQCRFYEALHDPNLNEEQRNARIRSIRDD-NH₂ GFNMQQQRRFYEALHDPNLNEEQRNARIRSIRDDC-NH₂ GFNMQQQRRFYEALHDPNLNEEQRNARIRSIRDDP-SRRK(Z)R-NH₂ GFNMQQQRRFYEALHDPNLNEEQRNARIRSIRDDP-SRRCR-NH₂ GFNMQQQCRFYEALHDPNLNEEQRNARICSIRDDP-SRRCRRCRRC-NH₂ GFNMQQQK(Z)RFYEALHDPNLNEEQRNARIK(Z)SIRDDP-SRRK(Z)RRK(Z)RRK(Z)-NH₂ FNMQCQZRFYEALHDPNLNEEQRNARIRSIRDDC-NH₂ Acetyl-KNMQCQZRFYEALHDPNLNEEQRNARIRSIRDDC-NH₂ GFNMQCQK(Z)RFYEALHDPNLNEEQRNARIRSIRDDC-SRRK(Z)R-NH₂ FNMQCQK(Z)RFYEALHDPNLNEEQRNARIRSIRDDC-NH₂ Acetyl-KNMQCQK(Z)RFYEALHDPNLNEEQRNARIRSIRDDC-SRRK(Z)R-NH₂ GFNMQCQK(Z)RFYEALHDPNLNEEQRNARIRSIRDDC-SRRK(Z)RRK(Z)RRK(Z)-NH₂ Acetyl-KNMQCQK(Z)RFYEALHDPNLNEEQRNARIRSIRDDC-SRRK(Z)RRK(Z)RRK(Z)-NH₂ GFNMQCQK(Z)RFYEALHDPNLNEEQRNARIRSIRDDC-SRRK(Z)RRK(Z)RRK(Z)-NH₂

Preferably, Fc-binding IgBP is one where the "functional group (Z)" for binding other functional molecule intended to be bound to an Fc molecule is bound to a lysine residue, the amino group at the N terminal, or a linker, or is bound to other moiety, for example, the C terminal via other group or linker. The "functional group" herein means a group that can be reacted with and bound to a peptide, a protein, a nucleic acid, or a low-molecular medicine under mild conditions. Examples of the functional group include maleimide, thiol or protected thiol, alcohol, acrylate, acrylamide, amine or protected amine, carboxylic acid or protected carboxylic acid, azide, alkynes including cycloalkyne, 1,3-dienes including cyclopentadiene and furan, alpha-halocarbonyl, N-hydroxysuccinimidyl, N-hydroxysulfosuccinimidyl, nitrophenyl ester, carbonate, dibenzocyclooctyne (DBCO), tetrazine, methyltetrazine (MTZ), and trans-cyclooctene (TCO).

For example, Fc-binding IgBP herein may be one where an azide group as a functional group is bound to the N terminal or C terminal (preferably N terminal), if necessary, via a linker.

Preferably, an azide group is contained at a terminal of a peptide where one to three (preferably two) glutamic acids are further bound to the N terminal and/or C terminal of IgBP. IgBP having an azide group can be subjected to a click reaction with other functional molecule having Dibenzylcyclooctyne (DBCO), alkyne, or TCO, to result in linkage of such other functional molecule to IgBP. Binding of Fc-binding IgBP and such other functional substance can also be performed by a method known by those skilled in the art, for example, a reaction of a maleimide group and a sulfhydryl group.

Other functional molecule may be bound to Fc-binding IgBP herein. For example, such other molecule can also be bound (for example, to an amino terminal) via the functional group. When an amino acid (for example, lysine residue) in the Fc-binding IgBP has a functional group, such other functional molecule can also be bound to the functional group (for example, an azide group as a substituent of a lysine residue) or can also be bound to a Cys residue (preferably Linker, for example, a Cys residue in Linker 2 or Linker 3) in the Fc-binding IgBP, via a maleimide group.

Such other functional molecule that can be bound to the Fc-binding IgBP encompasses, but not limited to, a labeling substance or a medical drug including a peptide, a protein, a nucleic acid, or a low-molecular medicine. Any molecule to which antigen specificity and other properties of an Fc molecule can be applied can be bound as such other molecule. Examples of such a substance include an anticancer drug, a low-molecular pharmaceutical product, a radioactive label, a fluorescent label, a nucleic acid medicine, a gene therapy agent, a peptide medicine, and an antibody such as IgA or VHH

When labeled with a labeling substance, the Fc-binding IgBP can be taken with an Fc molecule to form a composite and thus the Fc molecule can be detected or quantitatively determined with the labeling substance. Examples of the labeling substance include, but not limited to, fluorescent dyes, chemiluminescent dyes, radioactive isotopes (for example, chelate complex of radioactive iodine or radioisotope metal ion, for example, chelate complex of DOTA or desferrioxamine), and fluorescent proteins such as biotin and GFP (green fluorescent protein), luminescent proteins and enzymes such as peroxidase, and preferable examples of the labeling substance are fluorescein derivatives such as fluorescein and FITC, rhodamine derivatives such as rhodamine and tetramethylrhodamine, and fluorescent dyes such as Texas Red.

In the case of modification of the Fc-binding IgBP by other drug, examples of such other drug include, but not limited to, auristatin such as auristatin E, anticancer agents such as maytansine, emtansine, doxorubicin, bleomycin, or derivatives thereof; and targeting agents such as a drug to be bound to a receptor on the blood-brain barrier to allow for transfer to the central nerve, or a drug to be bound to a cancer cell to allow for transfer of an antibody into a cell. The Fc-binding IgBP, when linked to a drug, can be, for example, taken with IgG for use as a medicine antibody to form a composite, and thus can be enhanced in therapeutic effect of a disease.

The Fc-binding IgBP has at least one amino group (-NH₂), for the purpose of covalent binding with an antibody. Such an amino group may be the amino group at the amino terminal, or may be a side chain amino group of a lysine residue, a cysteine residue, an aspartic acid residue, a glutamic acid residue, a 2-aminosuberic acid, a diaminopropionic acid, or an arginine residue.

An amino group of a lysine residue, a cysteine residue, an aspartic acid residue, a glutamic acid residue, a 2-aminosuberic acid, a diaminopropionic acid, an arginine residue (preferably lysine residue) or an amino acid at the 1-position in the Fc-binding IgBP may be optionally modified by a crosslinking agent for covalent binding to an antibody. Herein, IgBP thus modified by the crosslinking agent may be sometimes referred to as "CCAP reagent". The Fc-binding IgBP is preferably the CCAP reagent. The "crosslinking agent" is herein a chemical substance for linking IgBP and an Fc molecule by a covalent bond. The crosslinking agent can be appropriately selected by those skilled in the art, and can be a compound having at least two sites that can be each bound to a desired amino acid (for example, a lysine residue, a cysteine residue, an aspartic acid residue, a glutamic acid residue, a 2-aminosuberic acid, diaminopropionic acid, or an arginine residue). Examples of the crosslinking agent can include DSG (disuccinimidyl glutarate), DSS (disuccinimidyl suberate), DMA (dimethyl adipimidate dihydrochloride), DMP (dimethyl pimelimidate dihydrochloride), DMS (dimethyl suberimidate dihydrochloride), DTBP (dimethyl 3,3'-dithiobispropionimidate dihydrochloride), and DSP (dithiobis(succinimidylpropionic acid)), and DSG, DSS, or DSP is preferable. For example, a crosslinking agent having a succinimidyl group such as DSS or DSG is reacted with a primary amine present in a side chain of a lysine residue or the N terminal of a polypeptide, and thus the N terminal of IgBP can be blocked and then reacted with DSS or DSG, to thereby allow only a side chain of a lysine residue of IgBP to be specifically modified by DSS or DSG. A combination of such an amino acid residue and the crosslinking agent can be appropriately selected by those skilled in the art. Crosslinking between IgBP modified by the crosslinking agent and IgG can be site-specifically produced between, for example, an amino acid residue of X⁵, X⁹, X¹¹, X¹², or X¹⁴ described above of IgBP and Lys248 or Lys246 in an Fc region of IgG, preferably Lys248.

Two cysteine residues, cysteine closest to the C terminal and cysteine closest to the N terminal, may be preferably linked via a disulfide bond or linker to thereby form a cyclic peptide in each of the peptides represented by the formulae (I), (I'), (I"), (II), and (II'). Examples of a divalent group available as such a linker can include a -CH₂-C(=O)-CH₂- group, a -CH₂-C(=O)- group, a -CH₂-C(=N-R)-CH₂- group, a -CH₂-O-CH₂- group, a -C(=O)- group, and a -C(=N-R)- group (wherein R is an alkyl group optionally substituted, for example, C1 to C6 alkyl, a methyl group, an ethyl group, a propyl group, a butyl group, a pentyl group, or a hexyl group, each optionally substituted), and a substituent in the case of substitution is, for example, a hydroxy group, a (mono or poly)ethylene oxide group, a carboxyl group, an alkoxy group, an acyl group, an alkyl group, an amide group, an ester group, a halogen group (F, Cl, Br, or I), or a combination of these groups). The linker is more excellent in stability, for example, alkali resistance or resistance to a reduction reaction or the like, preferably alkali resistance, than a usual disulfide bond. Examples of the two cysteine residues thus bound can include a cysteine residue located between X¹ and X² and a cysteine residue located between W and X⁸ in each of the formulae (I), (I'), and (I"), and, in the case of both X¹⁰ and X¹³ in each of the formulae (II) and (II') being cysteine residues, can include these cysteine residues.

Herein, K(Z) means a functional group-bound lysine residue, and preferably K(Z) is K(Azide).

The amino group of the amino acid at the N terminal of IgBP herein may be acetylated. The carboxyl group of the amino acid at the C terminal in IgBP herein may be amidated (to which an amino group may be bound). Furthermore, IgBP herein may be, if necessary, modified by PEG, at a terminal thereof or between the azide group and a peptide moiety. In the case of modification by PEG, the degree of polymerization of PEG can be 2 to 10, 3 to 8, or 4 to 7.

An Fc molecule contains an Fc region of IgG and the region has a pair of two symmetric partial peptide chains (Fc chains) in a heavy chain constant region, and thus two sites to which Fc-binding IgBP is bound are present. Thus, zero to two IgBPs for Fc molecule binding can be bound to one Fc molecule. The "Fc molecule where one IgBP is bound to one Fc molecule" means an Fc molecule where IgBP is bound to only one of the two symmetric partial peptides derived from a heavy chain constant region in an Fc molecule. Herein, binding of no Fc-binding IgBP to one Fc molecule is referred to as "zerovalent", binding of one Fc-binding IgBP to one Fc molecule is referred to as "monovalent", and binding of two IgBPs for Fc molecule binding to one Fc molecule is referred to as "divalent". Herein, the word "one Fc molecule" means a molecule containing a pair of two Fc regions that can constitute an IgG1 molecule.

In amino acid sequences described herein, A is an alanine residue, R is an arginine residue, N is an asparagine residue, D is an aspartic acid residue, C is a cysteine residue, Q is a glutamine residue, E is a glutamic acid residue, G is a glycine residue, H is a histidine residue, I is an isoleucine residue, L is a leucine residue, K is a lysine residue, M is a methionine residue, F is a phenylalanine residue, P is a proline residue, S is a serine residue, T is a threonine residue, W is a tryptophan residue, Y is a tyrosine residue, and V is a valine residue. Moreover, Hcy is homocysteine, Dpr is diaminopropionic acid, Orn is an ornithine residue, βAla is a β-alanine residue, Dab is a 2,4-diaminobutyric acid residue, Nle is a norleucine residue, Nva is a norvaline residue, Tie is a tert-leucine residue, Ala(t-Bu) is a tert-butylalanine residue, and Cha is a cyclohexylalanine residue. In each residue (a lysine residue, an ornithine residue, and a 2,4-diaminobutyric acid residue) having an amino group in a side chain, such an amino group may be, if necessary, acetylated. While each acetylated form of natural and artificial amino acids may also be herein designated with a prefix Ac in amino acid designation described above, it is understood that such an acetylated form can be included even if no Ac is designated, except in cases where an interpretation of the inclusion of such an acetylated form is inconsistent. K(Azide) represents an azide-bound lysine residue.

### (Method by purification)

In one aspect, the present disclosure relates to a method for increasing the proportion of a monovalent Fc molecule in an Fc molecule composition reacted with (Fc molecule-bound) IgBP, the method including
contacting an Fc molecule reacted with (bound to) Fc-binding IgBP, with a carrier to which Carrier-binding IgBP is bound, the IgBP being the same as or different from the Fc-binding IgBP, to thereby bind the Fc molecule bound to Fc-binding IgBP, to the carrier,
removing the Fc molecule not bound to the carrier, and
recovering the Fc molecule bound to the carrier.

The "Fc molecule composition reacted with Fc-binding IgBP" used in the purification method can be obtained by mixing Fc-binding IgBP and an Fc molecule according to any method described in International Publication Nos. WO 2008/054030, WO 2013/027796, WO 2016/186206, and WO 2018/230257. The method may include reacting Fc-binding IgBP and an Fc molecule to thereby obtain an Fc molecule composition reacted with Fc-binding IgBP, before the first step. Preferably, the "Fc molecule reacted with Fc-binding IgBP" means an Fc molecule covalently bound to Fc-binding IgBP through the above-mentioned crosslinking agent. When Fc-binding IgBP is modified by the crosslinking agent, conditions in the mixing step are not particularly limited as long as the step is performed in conditions that generate a crosslinking reaction between Fc-binding IgBP and an Fc molecule, and, for example, Fc-binding IgBP and an Fc molecule can be mixed and thus reacted in a proper buffer at room temperature (for example, about 15°C to 30°C). The mixing step may be performed by, if necessary, adding a proper amount of a catalyst that promotes the crosslinking reaction. The mixing ratio between Fc-binding IgBP and an Fc molecule in the mixing step is not particularly limited, and, for example, the molar ratio as IgBP:Fc molecule can be 1:1 to 20:1, preferably 2:1 to 20:1 or 5:1 to 10:1. The mixing time (reaction time) in the mixing step is not limited as long as the crosslinking reaction between Fc-binding IgBP and an Fc molecule is generated, and the mixing time can be, for example, 1 minute to 5 hours, preferably 10 minutes to 2 hours or 15 minutes to 1 hour. The Fc molecule composition reacted with Fc-binding IgBP may be, if necessary, further purified. The purification can be performed by a method known in the art, for example, chromatography such as gel filtration chromatography, ion exchange column chromatography, affinity chromatography, reverse phase column chromatography, and HPLC.

The present disclosure includes a "carrier to which Carrier-binding IgBP is bound". The carrier to which Carrier-binding IgBP is bound can be produced by, for example, reacting Carrier-binding IgBP with a carrier having a functional group reactive with an amino group. Examples of the form of the carrier include forms such as a gel (for example, gel for a column), particles, beads, nanoparticles, fine particles, macro beads, a membrane, a microplate, and an array, and examples of the material of the carrier include a magnetic substance, latex, agarose, glass, cellulose, sepharose, nitrocellulose, polystyrene, and other high molecular material. The carrier is preferably a gel for a column. The carrier here used can be, for example, HiTrap NHS-activated HP (GE Healthcare). The reaction is performed in conditions where both are sufficiently bound, and can be performed by, for example, contacting in a buffer at room temperature for 1 to 5 hours (preferably 2.5 to 3.5 hours).

The Fc molecule reacted with Fc-binding IgBP and the carrier to which Carrier-binding IgBP is bound are contacted in conditions where both can be sufficiently contacted. For example, when the carrier is a column, the Fc molecule reacted with Fc-binding IgBP is injected into a column in which Carrier-binding IgBP is immobilized.

The Fc molecule not bound to the carrier can be removed by an ordinary method, and can be removed by, for example, washing the carrier to which an Fc molecule is bound, with a buffer (pH: about 7.0).

The Fc molecule bound to the carrier can be recovered at a pH of 2.5 or more, and the acidity is desirably low in order to prevent the Fc molecule from being denatured, is preferably a pH of 3.6 or more and further preferably a pH of 3.7 or more, a pH of 3.8 or more in order to elute a monovalent Fc molecule modified, at a high proportion, can be a value at a pH of 3.8, 3.9, 4.0, 4.1, 4.2, or 4.3, or between any two of these values, and can be, for example, a pH of 3.6 to 4.3, a pH of 3.6 to 4.2, a pH of 3.6 to 4.1, a pH of 3.6 to 4.0, a pH of 3.7 to 4.2, a pH of 3.7 to 4.1, a pH of 3.7 to 4.0, a pH of 3.8 to 4.0, or a pH of 3.8 to 3.9.

The proportion of the monovalent Fc molecule relative to the entire Fc molecule (100%) in the Fc molecule composition recovered can be 55% or more, 63% or more, 70% or more, 80% or more, 90% or more, 95% or more, or 98% or more.

### (Method for binding IgBP on carrier)

In another aspect, the present disclosure relates to a method for preparing a monovalent Fc molecule by using Fc-binding IgBP and Carrier-binding IgBP, the method including
reacting an Fc molecule with a carrier to which Carrier-binding IgBP is bound, to thereby obtain an Fc molecule-bound carrier,
reacting the Fc molecule and Fc-binding IgBP in the obtained Fc molecule-bound carrier, on the carrier, to thereby obtain a bound product of (Fc-binding IgBP)-(Fc molecule)-(Carrier-binding IgBP)-(carrier), and
recovering a bound product of Fc-binding IgBP and the Fc molecule from the carrier, wherein
the Fc-binding IgBP and the Carrier-binding IgBP are optionally the same or different.

The present method may include a step of covalently binding an antibody and Fc-binding IgBP by a crosslinking agent by use of the Fc-binding IgBP as a CCAP reagent. The present method may further include a step of binding Fc-binding IgBP and other functional molecule. These steps may be each performed on the carrier before recovery of the bound product of Fc-binding IgBP and the Fc molecule from the carrier, or may be each applied to the bound product of Fc-binding IgBP and the Fc molecule, recovered from the carrier.

The carrier to which Carrier-binding IgBP is bound can be produced by the method described with respect to the above-mentioned purification method.

The reaction of the Fc molecule and the carrier to which Carrier-binding IgBP is bound can be performed by the method for contacting the Fc molecule reacted with Fc-binding IgBP and the carrier to which Carrier-binding IgBP is bound, described with respect to the above-mentioned purification method.

A reaction of a carrier-binding Fc molecule and Fc-binding IgBP can be performed by contacting both. The reaction temperature can be 4°C to room temperature, and the pH in the reaction can be 4.5 to 6.0. The reaction time is preferably 3 hours or more, more preferably 6 hours or more, or 12 hours or more. When the carrier is a column, the concentration of Fc-binding IgBP, to be used, can be 10 to 60 µM, or 20 to 40 µM. IgBP, preferably, Fc-binding IgBP is bound to a crosslinking agent, and the reaction of the Fc molecule and Fc-binding IgBP includes a crosslinking reaction.

The recovery of the bound product of Fc-binding IgBP and the Fc molecule from the carrier can be performed in the same manner as in the recovery of the Fc molecule bound to the carrier, described with respect to the above-mentioned purification method.

The proportion of the monovalent Fc molecule relative to the entire Fc molecule (100%) in the Fc molecule composition recovered can be 53% or more, 55% or more, 56% or more, 59% or more, 80% or more, 85% or more, 86% or more, or 89% or more.

In the method for preparing a monovalent Fc molecule, the proportion of the resulting monovalent Fc molecule relative to the entire Fc molecule (100%) used in the method can be 39% or more (or, 40% or more, 50% or more, 55% or more, or 74% or more).

When the carrier is a column, the present method can be automatized by using high-performance liquid chromatograph. In the case of automatization, the automatization can be performed under flow of a solution of 10 to 60 µM of Fc-binding IgBP at a rate of 0.03 to 1 mL/min or 0.05 to 1 mL/min for 10 minutes.

### (Method for preparing functional group-bound Fc molecule)

In one aspect, the present disclosure also encompasses a method for producing an Fc molecule to which a monovalent functional group or another functional molecule is introduced. Such a functional group or another functional molecule can be introduced via a binding group such as a functional group, a Cys residue, a Lys residue, or an amino group on IgBP, or can be introduced by binding IgBP and an Fc molecule and then covalently binding a binding group of the Fc molecule, such as a Cys residue or a Lys residue, and a compound having a functional group, or a functional molecule. The procedure may include binding IgBP for monovalent Fc molecule binding, to an Fc molecule, according to the above-mentioned method, and then binding a required functional group or another functional molecule, or using Fc-binding IgBP, to which such a functional group or another functional molecule is bound, to perform the above-mentioned method, thereby binding such Fc-binding IgBP, to which such a functional group or another functional molecule is introduced, to an Fc molecule.

It is also known that a functional group or other functional molecule can be covalently bound to and introduced into an Fc molecule by use of Fc-binding IgBP and thereafter such Fc-binding IgBP can be removed (Kei Yamada and others, (2019) Angew. Chem.; 131:5648-5653). In other words, after binding of Fc-binding IgBP, to an Fc molecule, a binding group such as a Cys residue or a Lys residue in the Fc molecule and a compound having a functional group, or a functional molecule may be covalently bound and Fc-binding IgBP may be dissociated from the Fc molecule, and then Fc-binding IgBP may be cleaved and removed from the Fc molecule. Such a step may be applied to a bound product of Carrier-binding IgBP and an Fc molecule on a carrier. Therefore, an Fc molecule to which a monovalent functional group or another functional molecule is introduced, of the present disclosure, may include no Fc-binding IgBP.

In other words, the present disclosure relates to a method for preparing an Fc molecule composition including a large amount of an Fc molecule (functional group-bound Fc molecule) where a functional group is bound to only one of two Fc chains present in one Fc molecule and where no IgBP is bound, the method including (i) reacting an Fc molecule with a carrier to which an IgG affinity peptide for carrier binding (Carrier-binding IgBP) is bound, to thereby obtain an Fc molecule-bound carrier; (ii) reacting an IgG affinity peptide for Fc molecule binding (cleavable crosslinking agent-bound IgBP: CCB-IgBP) to which a crosslinking agent is bound via a cleavable linker, with an Fc molecule in the Fc molecule-bound carrier obtained, to thereby obtain a bound product of CCB-IgBP and the Fc molecule; (iii) reacting and then covalently binding the crosslinking agent and the Fc molecule; (iv) cleaving the linker to cut a bond between the crosslinking agent and Fc-binding IgBP; (v) dissociating Fc-binding IgBP, from the Fc molecule, to thereby obtain a functional group-bound Fc molecule bound to the carrier via Carrier-binding IgBP, and free Fc-binding IgBP; and (vi) recovering the functional group-bound Fc molecule from the carrier; wherein the Fc-binding IgBP and the Carrier-binding IgBP are optionally the same or different; wherein a group containing a functional group is optionally present between the cleavable linker and the crosslinking agent; and wherein the functional group contained in the functional group-bound Fc molecule is a functional group present between the cleavable linker and the crosslinking agent (see FIG. 11E), or a group generated by linker cleavage (see FIG. 10C).

### (Method for preparing functional molecule-bound Fc molecule)

The present disclosure relates to a method for preparing an Fc molecule composition including a large amount of an Fc molecule (functional molecule-bound Fc molecule) where a functional molecule is bound to only one of two Fc chains present in one Fc molecule and where no IgBP is bound, the method including (i) reacting an Fc molecule with a carrier to which an IgG affinity peptide for carrier binding (Carrier-binding IgBP) is bound, to thereby obtain an Fc molecule-bound carrier; (ii) reacting an IgG affinity peptide for Fc molecule binding (cleavable crosslinking agent-bound IgBP: CCB-IgBP) to which a crosslinking agent is bound via a cleavable linker, with an Fc molecule in the Fc molecule-bound carrier obtained, to thereby obtain a bound product of CCB-IgBP and the Fc molecule; (iii) reacting and then covalently binding the crosslinking agent and the Fc molecule; (iv) cleaving the linker to cut a bond between the crosslinking agent and Fc-binding IgBP; (v) dissociating Fc-binding IgBP, from the Fc molecule, to thereby obtain a functional group-bound Fc molecule bound to the carrier via Carrier-binding IgBP, and free Fc-binding IgBP; (vi) binding a functional molecule to the functional group of the functional group-bound Fc molecule to thereby obtain a functional molecule-bound Fc molecule; and (vii) recovering the functional molecule-bound Fc molecule from the carrier; wherein the Fc-binding IgBP and the Carrier-binding IgBP are optionally the same or different; wherein a group containing a functional group is optionally present between the cleavable linker and the crosslinking agent; and wherein the functional group contained in the functional group-bound Fc molecule is a functional group present between the cleavable linker and the crosslinking agent (see FIG. 11E), or a group generated by linker cleavage (see FIG. 10C). Step (vi) of binding the functional molecule and step (vii) of recovering the Fc molecule from the carrier may be performed in any order in the present method.

The present disclosure relates to a method for preparing an Fc molecule composition including a large amount of a functional molecule-bound Fc molecule where a functional molecule is bound to only one of two Fc chains present in one Fc molecule and where no IgBP is bound, the method including (i) reacting an Fc molecule with a carrier to which an IgG affinity peptide for carrier binding (Carrier-binding IgBP) is bound, to thereby obtain an Fc molecule-bound carrier; (ii) reacting an IgG affinity peptide for Fc molecule binding (cleavable crosslinking agent-bound IgBP: CCB-IgBP) to which a crosslinking agent is bound via a cleavable linker, with an Fc molecule in the Fc molecule-bound carrier obtained, to thereby obtain a bound product of CCB-IgBP and the Fc molecule; (iii) reacting and then covalently binding the crosslinking agent and the Fc molecule; (iv) cleaving the linker to cut a bond between the crosslinking agent and Fc-binding IgBP; (v) dissociating Fc-binding IgBP, from the Fc molecule, to thereby obtain a functional molecule-bound Fc molecule bound to the carrier via Carrier-binding IgBP, and free Fc-binding IgBP; and (vi) recovering the functional molecule-bound Fc molecule from the carrier; wherein the Fc-binding IgBP and the Carrier-binding IgBP are optionally the same or different; and wherein a functional molecule is bound between the cleavable linker and the crosslinking agent (see FIG. 11E).

The "functional group-bound Fc molecule" means an Fc molecule where no IgBP is bound and a functional group is bound. The functional group may be bound to the Fc molecule via the linker, the crosslinking agent, and/or other structure. The "functional molecule-bound Fc molecule" means an Fc molecule where no IgBP is bound and a functional group is bound. The functional molecule may be bound to the Fc molecule via the linker, the crosslinking agent, and/or other structure. "CCB-IgBP" may include other substance as long as there is a structure where the crosslinking agent and IgBP are cut by decomposition of the cleavable linker, namely, binding is formed in the order of (crosslinking agent)-(cleavable linker)-(IgBP), and, for example, binding may be formed in the order of (crosslinking agent)-(substance or structure intended to be introduced into Fc molecule)-(cleavable linker)-(IgBP). A typified example of the substance or structure intended to be introduced into an Fc molecule is a group containing the functional molecule or functional group.

The "cleavable linker" herein refers to a linker having a binding group which is to be decomposed by acidic conditions, basic conditions, reduction, oxidation, enzyme treatment, light irradiation, or β dissociation to lead to cutting of a bond between the IgG affinity peptide for Fc molecule binding and the crosslinking agent, and examples can include a disulfide bond, an acetal bond, an ester bond, and an amide bond. One example of such a cleavable linker is described as a cutting portion in WO 2018/199337.

### (Composition)

In another aspect, the present disclosure relates to a composition including an IgBP-bound Fc molecule obtained according to the purification method or the preparation method. In other words, the present disclosure relates to an Fc molecule composition, in which the proportion of an Fc molecule to which only one IgBP is bound, in the entire Fc molecule, is higher than the corresponding proportion with respect to an IgBP-bound Fc molecule synthesized by a conventional CCAP method. A conventional CCAP method is a CCAP method including binding IgBP and an Fc molecule in a test tube, and the word is used for the purpose of distinction from the method using an IgBP-bound carrier, as described in the present disclosure. More specifically, a conventional CCAP method encompasses methods described in International Publication Nos. WO 2008/054030, WO 2013/027796, and WO 2016/186206.

A composition of the present disclosure preferably includes an Fc molecule to which only one IgBP is bound, at a proportion of 52% or more, 53% or more, 55% or more, 56% or more, 59% or more, 63% or more, 70% or more, 80% or more, 85% or more, 86% or more, 89% or more, 90% or more, 95% or more, or 98% or more, under the assumption that the entire Fc molecule is 100%.

The composition of the present disclosure can be, if necessary, a medical or diagnostic composition. When the composition of the present disclosure is a medical composition (therapeutic and/or preventive composition), IgBP is preferably modified by, for example, the above drug, and when the composition of the present disclosure is included in a diagnostic composition, IgBP is preferably modified by, for example, the above labeling substance. A disease to which the medical or diagnostic composition is directed can be appropriately set by selecting an Fc molecule to be used or a drug to be bound, and is preferably, for example, a disease or disorder that can be targeted by an antibody, and examples include cancer, inflammatory disease, infection, and neurodegenerative disease.

For example, the medical or diagnostic composition can be utilized as an injection product, and encompasses dosage forms such as an intravenous injection product, a subcutaneous injection product, an intradermal injection product, an intramuscular injection product, and an infusion injection product. Such an injection product can be prepared by, for example, dissolving, suspending or emulsifying an IgBP-bound Fc molecule in a sterile aqueous or oily liquid for use in a usual injection product, according to a known method. An injection solution prepared is usually packed in an appropriate ampule, vial, or syringe. A freeze-dried formulation can be prepared by adding an appropriate excipient to an IgBP-bound Fc molecule, and, when used, is dissolved in an injection solvent, saline or the like, and formed into an injection solution. A protein such as an antibody, although is generally broken down by the digestive organ and thus is difficult to orally administer, can be orally administered by inventiveness of an antibody fragment, an antibody fragment modified, and a dosage form. Examples of a formulation for oral administration can include a capsule, a tablet, a syrup, and a granule.

The medical or diagnostic composition is suitably prepared in a dosage form as a dose unit adaptable to the amount of an active ingredient administered. The dosage form as a dose unit is, for example, an injection product (an ampule, a vial, or a pre-filled syringe), and may usually contain 5 to 500 mg, 5 to 100 mg, 10 to 250 mg of an IgBP-bound Fc molecule per dosage form as a dose unit.

The medical or diagnostic composition may be locally or systematically administered. The administration method is not particularly limited, and is parenterally or orally administered as described above. Examples of a parenteral administration route include a subcutaneous or intraperitoneal injection or infusion, or an injection or infusion into the blood (intravenous, or intra-arterial) or the spinal fluid, and administration into the blood is preferable. The medical or diagnostic composition may be temporarily administered, or may be sustainably or interruptedly administered. For example, the administration can also be sustained administration for 1 minute to 2 weeks.

The administration of the medical or diagnostic composition is not particularly limited as long as the amount of the administration and the timing of the administration are adopted so as to lead to a desired therapeutic effect or preventive effect, and the administration can be appropriately determined depending on the symptom, sex, age, and the like. For example, administration by an intravenous injection is advantageous, which is usually made at a single dose of an active ingredient, of about 0.01 to 20 mg/kg body weight, preferably 0.1 to 10 mg/kg body weight, further preferably 0.1 to 5 mg/kg body weight, once to ten times per month, preferably once to five times per month, before and/or after development of a clinical symptom of the above disease. Also in the case of other parenteral administration and oral administration, such administration can be made at a dose corresponding to the above dose.

Hereinafter, the present disclosure is described in more detail with reference to Embodiments, but these Embodiments are not intended to limit the scope of the present disclosure. Literatures cited throughout the description of the present application are herein incorporated by reference in their entireties.

### (Materials)

A protein A column, a protein G column, and a column for peptide immobilization here used were respectively HiTrap Protein A HP Columns, HiTrap Protein G HP, and a HiTrap NHS-activated HP (1 ml, GE Healthcare) column.
Embodiment 1: Serum half-life of antibody conjugate according to CCAP method (1) Production of antibody conjugate according to CCAP method
To 5 ml of an 8 µM anti-AVM (avermectin) antibody (human IgG4) dissolved in a 20 mM acetate buffer (pH 4.5) was 154 µL of CCAP reagent Azide-PEG4-EEGPDCAYH (succinimidyl glutaryl Lys) GELVWCTFH (SEQ ID No. 69)-NH₂ (Azide-EEIgBP, 534 µM) dissolved in DMSO (final concentration 16 µM), and the resulting solution was reacted at room temperature for 1 hour. A portion (antibody 3 µg) of the solution was injected into anion exchange column Shodex QA825 (Shodex) connected to Nexera-i (Shimazu), and was subjected to NaCl gradient elution (0 to 1 M) in a 10 mM Tris buffer (pH 8.0). The results are shown in FIG. 1. The resulting monovalent antibody and divalent antibody were separately collected, dialyzed by PBS, and then used as a sample for measurement of serum half-life measurement.

### (2) Measurement of serum half-life

Mice were used for measurement of each serum half-life of a monovalent antibody and a divalent antibody each obtained by modifying an anti-avermectin (AVM) antibody by an Azide-EEIgBP peptide reagent. An control antibody (AVM), a monovalent peptide antibody (AVM-pep1), and a divalent peptide antibody (AVM-pep2) were each i.v. administered in an amount of 5 mg/kg to ICR mice (three mice per group), and the antibody concentrations in serums were analyzed after 1 hour, 4 hours, 24 hours, 48 hours, 72 hours, and 168 hours.

### (3) Results

FIG. 2 illustrates the results of the antibody concentrations in serums, analyzed after 1 hour, 4 hours, 24 hours, 48 hours, 72 hours, and 168 hours of antibody administration. Table 1 shows the pharmacokinetic parameters analyzed from the change in antibody concentration in serum in the experiment. It was found as shown in Table 1 that the half-life (T1/2β = 264 h) of the monovalent antibody was almost equivalent to that (T1/2β = 268 h) of the unmodified antibody, whereas the half-life (T1/2β = 139 h) of the divalent antibody was about half that of the unmodified antibody. In other words, it was found that, while monovalent modification did not have a large effect on the serum half-life, divalent modification caused the serum half-life of the modified antibody to be significantly decreased.

### [Table 1]

**Table 1 Parameters of mouse serum half-lives of monovalent antibody and divalent antibody**

| | t_{1/2α} (h) | t_{1/2β} (h) | AUC₀₋ₜ (µg·h/mL) | AUC_{0-∞} (µg·h/mL) | CL (L/h/kg) | V_{dss} (L/kg) | MRT (h) |
|---|---|---|---|---|---|---|---|
| AVM | 11.6±2.3 | 284±144 | 6490±490 | 18000±7400 | 0.305±0.102 | 107±14 | 394±205 |
| AVM peptide monovalent | 9.29+3.43 | 264±85 | 5350+750 | 14200±4400 | 0.382±0.143 | 129±18 | 367±121 |
| AVM peptide divalent | 7.54±0.98 | 139±33 | 1720±320 | 2500±520 | 2.06±0.42 | 297±82 | 146±39 |

In the case of use of a conjugate antibody produced according to a CCAP method, as a pharmaceutical product, use of a monovalent modified body having the same serum half-life as in an unmodified body is important also for retaining of biological activities of a drug. However, in the case of production of a conjugate according to a CCAP method, a divalent antibody is necessarily generated in addition to a monovalent antibody also as illustrated in FIG. 1. It has been then found that there is a need for a purification procedure of a monovalent antibody at a high purity by increasing the efficiency of generation of a monovalent antibody with suppression of generation of a divalent antibody at the stage of a generation reaction, or efficiently removing a divalent antibody.

### Embodiment 2: Production of antibody binding column

### (1) Production of IgBP-immobilized column

IgG-bound peptide IgBP (WO 2013/027796): NH₂-GSGGS-DCAYHRGELVWCT-CONH₂ (MW: 1895.06) (SEQ ID No. 70) where a GS linker was added to the N terminal was obtained by solid-state synthesis by use of an F-moc method, and deprotected and then purified by reverse phase HPLC. 14 mg of the peptide freeze-dried was dissolved in 0.25 mL of a 50 mM Tris buffer (pH 8.6), and the resulting solution was left to still stand at room temperature for 1 hour. After formation of an SS bond was confirmed by LC-MS (SQ detector with Acquity UPLC connected, Waters), TFA was added so as to be at a final percentage of 1%, leading to acidity. The solution was applied to a reverse phase column (Sep-Pak C18), washed with 0.1% TFA, then eluted with 60% acetonitrile containing 0.1% TFA for removal of an organic solvent, and thereafter freeze-dried.

After 0.720 mL of a solution of 7.5 mM peptide dissolved in DMSO was diluted with 2.88 mL of 0.2 M NaHCO₃ (pH = 8.3), the diluted solution was slowly injected in each amount of 1 ml (amount of peptide: 1.5 µmol) into HiTrap NHS-activated HP (1 ml, GE Healthcare) columns (three columns) each washed with 5 mL of 1 mM HCl, and left to still stand at room temperature for 3 hours after the injection. After washing with 5 mL of 0.2 M NaHCO₃ (pH = 8.3), blocking (10 minutes) with 0.5 M monoethanolamine-HCl (pH 7.5) containing 6 mL of 0.5 M NaCl and washing with 0.1 M sodium acetate-HCl buffer (pH 4.0) containing 6 mL of 0.5 M NaCl were alternately repeated three times. Finally, each of the columns was flushed with 5 mL of a 20 mM phosphate buffer (pH 7.0), and stored at 4°C. The respective amounts of peptides immobilized to the three columns thus produced were evaluated to be 72.7% (1090.5 nmol), 77.4% (1161 nmol) and 73.1% (1096.5 nmol) in terms of the proportion relative to the peptide administered.

### (2) Production of Z33-immobilized column

IgG-bound peptide Z33 (Braisted, A.C. and others, Proc. Natl. Acad. Sci. U.S.A. 93: 5688-92 (1996)): Acetyl-FNMQQQRRFYEALHDPNLNEEQRNARIRSIRDDPSGSGSK-NH₂) (SEQ ID No. 71) where a linker moiety for column immobilization was introduced to the C terminal was obtained by solid-state synthesis by use of an F-moc method, and deprotected and then purified by reverse phase HPLC. A 15 mM solution was prepared by dissolving 23.8 mg of the peptide freeze-dried, in 330 µL of DMSO. DMSO was added in the same amount to 250 µL of the peptide solution, and the resulting solution was diluted with 2.0 mL of 0.2 M NaHCO₃ (pH = 8.3), thereafter loaded in each amount of 1 ml (amount of peptide: 1.5 µmol) to HiTrap NHS-activated HP (1 ml, GE Healthcare) columns (two columns) each washed with 5 mL of 1 mM HCl, and left to still stand at room temperature for 3 hours. After washing with 5 mL of 0.2 M NaHCO₃ (pH = 8.3), blocking (10 minutes) with 0.5 M monoethanolamine-HCl (pH 7.5) containing 6 mL of 0.5 M NaCl and washing with 0.1 M sodium acetate-HCl buffer (pH 4.0) containing 6 mL of 0.5 M NaCl were alternately repeated three times. Finally, each of the columns was flushed with 5 mL of 20 mM phosphate buffer (pH 7.0), and stored at 4°C. The respective amounts of peptides immobilized to the two columns thus produced were evaluated to be 60.8% (912 nmol) and 40.5% (608 nmol) in terms of the proportion relative to the peptide administered.

### (3) Elution conditions of IgBP-immobilized column and evaluation of DBC (Dynamic binding capacity)

A 1-ml column (amount of IgBP immobilized: 995 nmol) where IgBP was immobilized was connected to NGC^{™} chromatography system (BioRad), and equilibrated by flowing of a 20 mM phosphate buffer (pH 7.0) at 2 mL/min for 5 minutes. One mg of a human IgG1 antibody dissolved in 50 mL of 20 mM phosphate buffer (pH 7.0) was injected, washed with phosphate buffer (pH 7.0) for 3 minutes, and thereafter subjected to first elution with elution of 0.1 M glycine hydrochloride at each pH of 3.5, 3.0 and 2.5 for 2.5 minutes. Furthermore, the second elution was performed by 0.1 M glycine hydrochloride at a pH of 2.5, and normalization by 20 mM phosphate buffer (pH 7.0) was finally performed (FIG. 3 and FIG. 4).

FIG. 4 includes enlarged diagrams in the first elution. While the elution time tended to be earlier as the pH was lowered from 3.5 to 3.0 and 2.5, almost no difference in peak area of an antibody eluted was found among the pH values and no elution was observed at the second elution at a pH of 2.5, and it was thus found that antibody elution from the column was sufficiently performed at a pH of 3.5.

Next, the same column was used to evaluate dynamic binding capacity (DBC). The column, which was connected to NGC^{™} chromatography system (BioRad), was equilibrated by flowing of a 20 mM phosphate buffer (pH 7.0) at 1.0 mL/min for 10 minutes, and 30 mL of a solution of human serum-derived IgG (SIGMA, #14506, Lot# SLBR0560V) dissolved in the buffer so that 0.98 mg/ml was achieved was injected to the column at 1 mL/min. DBC was determined from the midpoint of leakage from the column, and thus was found to be 47 mg. After washing with buffer at 1 mL/min for 6 minutes, IgG bound was eluted with 0.1 M glycine hydrochloride (pH 3.5) at a flow rate of 2.0 mL/min, and the amount of the antibody bound to the column was calculated from the absorbance of A280 of protein after elution and was found to be 42 mg (the amount of the whole IgG antibody was calculated under the assumption of 1 mg/mL = Abs 280:1.38).

### (4) Elution conditions of Z33-immobilized column and evaluation of dynamic binding capacity (DBC)

Elution conditions of an antibody by a Z33-immobilized column produced were examined. A 1-ml column (amount of IgBP immobilized: 912 nmol) where Z33 was immobilized was connected to NGC^{™} chromatography system (BioRad), and equilibrated by flowing of a 20 mM phosphate buffer (pH 7.0) at 1 mL/min for 10 minutes. One mg of a human IgG1 antibody dissolved in 10 mL of 20 mM phosphate buffer (pH 7.0) was injected, washed with phosphate buffer (pH 7.0) for 6 minutes, and thereafter eluted at a flow rate of 2 mL/min with an elution liquid of 0.1 M glycine hydrochloride at each pH of 2.5 and pH 3.5 (FIG. 5). A favorable elution behavior was observed in each elution at both the pH values, and it was thus found that antibody elution from the column was sufficiently performed at a pH of 3.5.

Next, the same column was used to evaluate dynamic binding capacity(DBC). The column, which was connected to NGC^{™} chromatography system (BioRad), was equilibrated by flowing of a 20 mM phosphate buffer (pH 7.0) at 1.0 mL/min for 10 minutes, and 50 mL of a solution of human serum-derived IgG (SIGMA, #14506, Lot# SLBR0560V) dissolved in the buffer so that 0.67 mg/ml was achieved was injected to the column at 1 mL/min. DBC was determined from the midpoint of leakage from the column, and thus was found to be 26 mg. After washing with buffer at 2 mL/min for 3 minutes, IgG bound was eluted with 0.1 M glycine hydrochloride (pH 3.5) at a flow rate of 2.0 mL/min, and the amount of the antibody bound to the column was calculated from the absorbance of A280 of protein after elution and was found to be 19 mg (the amount of the whole IgG antibody was calculated under the assumption of 1 mg/mL = Abs 280:1.38).

### (5) Analysis of CCAP modified body on column by ion exchange column

An antibody recovered as a washing fraction or an elution fraction from an affinity column was analyzed by injecting 0.05 mL of each solution to cation exchange column Shodex SP825 (Shodex) connected Nexera-i (Shimazu), and performing NaCl gradient elution (0 to 0.3 M) in a 10 mM acetate buffer (pH = 5.5).

### Embodiment 3: Purification of monovalent antibody by use of IgBP-immobilized column

### (1) Removal of divalent antibody by use of IgBP-immobilized column

10 mg (67 nmol) of Trastuzumab was dissolved in 3.3 ml of a 20 mM phosphate buffer (pH 7.0), 2.9 µL (202 nmol) of a 70 mM CCAP reagent (MTZ-DBCO-N₃-(PEG)₇-RRDCAYHKGELVWCT (SEQ ID No. 72)-NH₂) dissolved in DMSO was added, and a reaction was made at room temperature overnight. A portion (about 3 µg) was taken out, injected to Simadzu LC2040 (Shimazu) to which hydrophobic chromatography column TSKgel Butyl-NPR (4.6 mm I.D. × 10 cm, Tosoh) was connected, and eluted at a flow rate of 0.5 ml/min in a gradient from an A liquid (25 mM phosphate buffer (pH 7.0) containing 1.5 M ammonium sulfate) to a B liquid (25 mM phosphate buffer (pH 7.0) containing 20% isopropanol) for 20 minutes (Section (A) of FIG. 6). The remaining reaction product was injected to an IgBP-immobilized column, washed with 20 mM phosphate buffer (pH 7.0), and thereafter eluted by a 0.1 M glycine hydrochloride buffer (pH 3.5). The analysis results by hydrophobic chromatography, of a fraction flowing through the column and a fraction eluted in an acidic solution from the column are illustrated in sections (B) and (C) of FIG. 6. It was found that the IgBP column enabled only an unmodified body and a monovalent antibody to be purified by allowing a divalent antibody present in a reaction product of IgG and the CCAP reagent to flow through the IgBP column and thus be removed (Section (C) of FIG. 6).

### (9) Removal of divalent antibody from reaction product according to CCAP method by use of Z3 3 -immobilized column

A portion (about 3 µg) of a sample prepared by the same method as in (8) described above was analyzed by a hydrophobic chromatography column (Section (A) of FIG. 7). The remaining reaction product was injected to the Z33-immobilized column, washed with a 20 mM phosphate buffer (pH 7.0), and thereafter eluted with a 0.1 M glycine hydrochloride buffer (pH 3.5). The analysis results by hydrophobic chromatography, of a fraction flowing through the column and a fraction eluted in a solution having a pH of 3.5 from the column are illustrated in sections (B) and (C) of FIG. 7. It was found that the Z33 column also enabled only an unmodified body and a monovalent antibody to be efficiently purified by allowing a divalent antibody in a reaction product of IgG and the CCAP reagent to flow through the IgBP column and eluting an antibody adsorbing to the column with an acidic solution, as in the IgBP column.

### (10) Purification of monovalent antibody by pH control in elution

As described above, the IgBP column enabled a divalent antibody and a mixture of a monovalent antibody and an unmodified antibody to be separated, but did not enable a monovalent antibody and an unmodified antibody to be separated. A monovalent antibody and an unmodified antibody were tried to be separated by pH control in elution. In other words, Trastuzumab and a CCAP reagent (IgBP-N₃-RRRSS-SG) were reacted at a molar ratio of 1:1 at room temperature for 2 hours, and thereafter a reaction product was applied to the IgBP column equilibrated by a 0.1 M acetate buffer (pH 5.5), washed with the buffer, and thereafter subjected to first elution with a 0.1 M acetate buffer (pH 4.0, pH 3.9, pH 3.8, pH 3.7) or a 0.1 M glycine hydrochloride buffer (pH 3.6) and then second elution with a 0.1 M glycine hydrochloride buffer (pH 3.5).

As illustrated in FIG. 8A, each elution peak was observed in all conditions of pH values of 3.6 to 4.0 in first elution, and no peak was observed only in the case where elution was performed at a pH of 3.6 in the first elution, in the second elution.

FIG. 8B illustrates the results of ion exchange chromatography of each fraction in the first elution at a pH of 3.8, and it has been found that a divalent antibody, a monovalent antibody and a divalent antibody are respectively eluted as main components in a flow-through fraction, an elution fraction at the first stage, and an elution fraction at the second stage, and such components can be clearly separated by the IgBP column. The contents of zerovalent, monovalent, and divalent antibodies in respective conditions are summarized in Table 2. It has been found that, while the purity is slightly different among conditions of pH values of 4.0 to 3.7 in the first elution, a monovalent antibody can be purified at a high purity of 95% or more in all such conditions. In this regard, it has been found that a zerovalent antibody and a monovalent antibody are simultaneously eluted and cannot be separated at a pH of 3.6 in the first elution.

### [Table 2]

**Table 2 Content ratios (%) of zerovalent, monovalent, and divalent antibodies in flow-through fraction, fraction in first elution (pH 4.0, 3.9, 3.8, 3.7, 3.6), and fraction in second elution (pH 3.5) of reaction product of Trastuzumab and CCAP reagent from IgG-BP column, by ion exchange chromatography analysis**

| pH in first elution | application (%) | | | First elution (%) | | | Second elution (%) | | |
|---|---|---|---|---|---|---|---|---|---|
| | Zerovalent | Monovalent | Divalent | Zerovalent | Monovalent | Divalent | Zerovalent | Monovalent | Divalent |
| 4 0 | 0 | 0 | 100 | 1.7 | 98.7 | 0 | 89.1 | 10.9 | 0 |
| 3.9 | 0 | 0 | 100 | 2.2 | 97.7 | 0 | 94.1 | 5.9 | 0 |
| 3.8 | 0 | 0 | 100 | 3.6 | 96.4 | 0 | 97.2 | 2.8 | 0 |
| 3.7 | 0 | 0 | 100 | 4.6 | 95.4 | 0 | 98.2 | 1.8 | 0 |
| 3.6 | 0 | 0 | 100 | 36.4 | 63.6 | 0 | - | - | - |

### Embodiment 4: Preparation of monovalent antibody in CCAP reaction on IgBP-immobilized column, or Protein A or Protein G column

It has been found from the previous section that the IgBP column can be used to purify a monovalent antibody at a high purity of 95% or more by pH control in elution. However, generation of a divalent antibody due to a reaction was difficult to suppress in a solution and thus only at most more than 50% of a monovalent antibody, relative to the antibody subjected to the reaction, could be recovered in terms of the yield observed based on the total. Thus, examined was a procedure for preferentially synthesizing a monovalent antibody with suppression of generation of a divalent antibody, by reacting an antibody with a CCAP reagent in the state of once adsorption of the antibody to an IgBP column, or a Protein A or Protein G.

1 mg of Trastuzumab was diluted with 10 mL of a 0.1 M acetate buffer (pH 5.5), and injected to an IgBP column equilibrated by the buffer. Thereafter, a CCAP reagent solution diluted with a buffer to 20 or 60 µM at the last minute was allowed to flow in a 1-ml column. The column was subjected to incubation at room temperature or 4°C for 3 hours or 12 hours, and washed with 5 ml of a 0.1 M acetate buffer (pH 5.5), and thereafter elution with 5 mL of a 0.1 M glycine hydrochloride buffer (pH 3.5) was performed. A flow-through fraction recovered from a reaction product in each condition, and an elution fraction were analyzed by cation exchange column chromatography used in Example 2, and the proportions (%) of zerovalent, monolavent, and divalent antibodies contained in each of the fractions were calculated. The results are shown in Table 3 (which shows only the content proportion of a divalent antibody because of recovery of only the divalent antibody from a washing liquid).

Among Conditions 1 to 4 in Table 3, a case of a reaction of IgG immobilized on the IgBP column in a concentration of a CCAP reagent, of 20 µM, at room temperature for 12 hours (Condition 2) exhibited the highest proportion of a monovalent antibody among Conditions compared, in which the rate of generation of a monovalent antibody included in an elution fraction was 74% based on the entire (flow-through fraction + elution fraction). In the case, no divalent antibody was included in the elution fraction, but 14% of a divalent antibody was observed in a washing fraction. In a case where the concentration of the CCAP reagent was threefold 60 µM in order to increase the rate of generation of a monovalent antibody (Condition 3), the proportion of a monovalent antibody in an elution fraction was 89% (the balance, 11%, corresponded to a zerovalent antibody) which was the highest proportion. In the case, the proportion of a divalent antibody in a flow-through fraction was increased to 56%, and the proportion of a monovalent antibody based on the entire remained at 39%. In this regard, the reaction time was shortened from 12 hours to 3 hours in order to reduce the proportion of a divalent antibody in a washing fraction, and thus the proportion of a divalent antibody eluted in a flow-through fraction was as low as 7% as intended, but the proportion of a monovalent antibody in an elution fraction was 59% (the balance, 41%, corresponded to a zerovalent antibody) and was lowered as compared with Conditions 2 (86%) and 3 (89%). A case where the reaction temperature was changed from room temperature to 4°C with the reaction time being 12 hours as it was, under the same objective (Condition 4) exhibited a decrease in proportion of a divalent antibody in a flow-through fraction, to 2%, as intended, and was indicated to be able to achieve a proportion of a monovalent antibody based on the entire, of 55% (the proportion in an elution fraction was also 56%).

A protein A column and a protein G column were each used as a commercially available column having the same function as in IgBP, and the same reaction on each of the columns was performed in Comparative Embodiments. The results were shown in Table 3. The binding sites of protein A and protein G to an antibody were almost the same as in IgBP and in a boundary region between CH2 and CH3 of Fc, and it was considered that generation of a divalent antibody by a CCAP reagent could be suppressed according to the same mechanism. A reaction using such a column was characterized in that a divalent antibody observed in the IgBP column was not observed in a flow-through fraction (Conditions 5 to 9). The reason was considered because protein A and protein G each had a ligand where the number of binding sites on an antibody was not one (for example, proteins A and G each had a binding site also in a Fab region) unlike IgBP, and thus could be continued to be bound to a column even if a divalent antibody by a CCAP reaction was generated in the column. This was also supported by inclusion of not only zerovalent and monovalent antibodies, but also a divalent antibody in each elution fraction at a pH of 3.5 from the protein A and G columns (Conditions 5 to 9 in Table 2). The purpose of use of the IgBP column in purification was to remove a divalent antibody from a recovered product in elution, and thus the protein A and G columns were found to be unsuitable for preparation of a monovalent antibody by the CCAP reaction on the column.

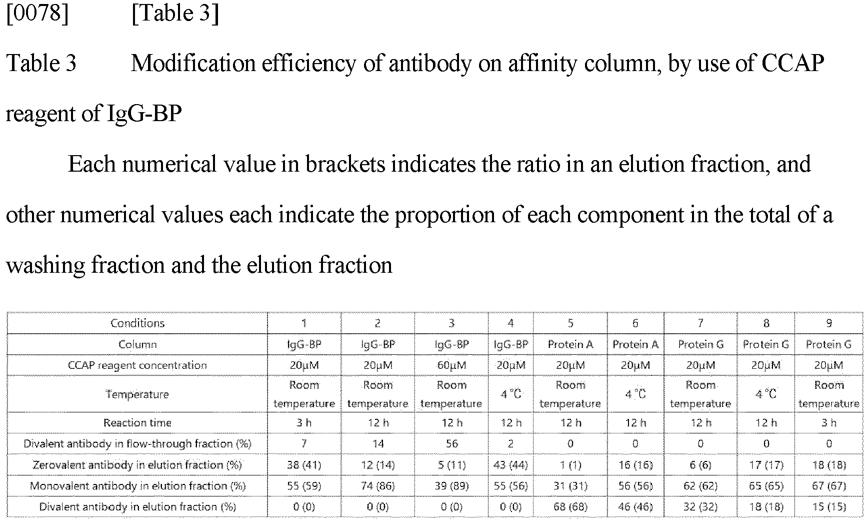

### Embodiment 5: Automotive CCAP reaction on IgBP column connected to high-performance liquid chromatograph

One solution for increasing reproducibility of production of a conjugate by a CCAP reaction is to automatize the reaction. The yield of each modified antibody by the CCAP reaction was examined with an IgBP column connected to high-performance liquid chromatograph (NGC, BioRad), according to a flow chart of FIG. 9A. An automotive CCAP reaction was performed in the following process with NGC (BioRad) chromatograph.
1) Injection of mAb: dissolving 1 mg of an antibody in 20 mL of a 0.1 M acetate buffer (pH 5.5), and injecting the resulting solution at 1 mL/min through S1 Port 1 (20 minutes).
2) Washing: washing a column by flowing of a 0.1 M acetate buffer (pH 5.5) at 1 mL/min for 10 minutes (10 minutes).
3) Reagent: injecting 1 mL of each CCAP reagent (10, 20, 30, 40 µM IgBP N₃RRR-SG) diluted with a 0.1 M acetate buffer (pH 5.5), at 0.1 mL/min (10 minutes).
4) Washing: washing the column by flowing of a 0.1 M acetate buffer (pH 5.5) at 1 mL/min for 10 minutes.
5) Elution: eluting a bound product from the column by flowing of a 0.1 M glycine hydrochloride buffer (pH 3.5) at 1 mL/min for 10 minutes.
6) Regeneration: regenerating the column by flowing of a 0.1 M acetate buffer (pH 5.5) at 1 mL/min for 12 minutes.

Each component included in an elution fraction was analyzed by the same method as in Example 4 (FIG. 9B). Only a trace of a monovalent antibody generated could be detected in a 10 µM reagent, but generation of the monovalent antibody (the balance corresponding to a zerovalent antibody) at a proportion of 52 to 59% in the elution fraction was confirmed in the case of addition of a 20 to 40 µM CCAP reagent. The concentration of the reagent was further increased to 60 µM, but the rate of generation of the monovalent antibody was not increased and was 57.8%. On the other hand, the contacting time of a reaction reagent and an antibody in the experiment was 10 minutes and was short as compared with the experiments in Tables 1 and 2. The rate of injection of the CCAP reagent was set at a flow rate of 0.05 mL/min and the contacting time was elongated twice, but the rate of generation of the monovalent antibody was also not increased and was 53.9% (no data shown in both cases). The rate of generation of a divalent antibody was actually 2% or less relative to the entire antibody recovered under the conditions, and it was considered that a significant loss in yield was not caused.

The foregoing results indicated that a monovalent antibody was obtained at a proportion of 52 to 59% by an automotive CCAP reaction using an IgBP column.

## Claims

1. A method for increasing a proportion of an Fc molecule where one Fc-binding IgBP, per Fc molecule, is bound, in an Fc molecule reacted with Fc-binding IgBP, the method comprising:
contacting an Fc molecule reacted with Fc-binding IgBP, with a carrier to which Carrier-binding IgBP is bound, the IgBP being the same as or different from the Fc-binding IgBP, to thereby bind the Fc molecule reacted with Fc-binding IgBP, to the carrier;
removing the Fc molecule not bound to the carrier; and
recovering the Fc molecule bound to the carrier.

2. The method according to claim 1, wherein the Fc molecule is IgG or an Fc fusion protein.

3. The method according to claim 1 or 2, wherein the Carrier-binding IgBP is the following peptide (i) or (ii):
(i) a peptide represented by the following formula (I):
NH₂-(Linker)-(X¹₁₋₃)-C-(X²)-(X³)-(X⁴)-(X⁵)-G-(X⁶)-L-(X⁷)-W-C-(X⁸₁₋₃) ... (I)
in the formula (I), (Linker) represents a linker, one to three X¹(S), X², X³, X⁴, X⁵, X⁶, X⁷, and one to three X⁸(s) each mutually independently represent the same or different amino acid residue,
each X¹, X², X³, and each X⁸ mutually independently represent any amino acid residues that are the same or different and are other than C,
X⁴ is H, R, S, or D,
X⁵ is one amino acid residue selected from K, C, D, E, R, V, F, L, 2-aminosuberic acid, Dpr, Orn, AcOrn, AcDab, Dab, Nle, Nva, Tie, Ala(t-Bu), and Cha,
X⁶ is E, N, R, or D, and
X⁷ is I or V; or
(ii) a peptide represented by the following formula (II), or a peptide containing an amino acid sequence of (II), where one or several amino acids are each added, deleted, and/or substituted at a position other than X⁹ to X¹³:
X⁹₁₋₂NMQX¹⁰QRRFYEALHDPNLNEEQRNAX¹¹IX¹²SIRDDX¹³-(Linker 2)-CONH₂ (SEQ ID No. 60) ... (II)
in the formula (II), (Linker 2) represents a linker, X⁹₁₋₂ is selected from the group consisting of GF, AF, VF, LF, IF, MF, PF, FF, WF, KF, Orn-F, CF, DF, EF, βAla-F, 2-aminosuberic acid-F, Dpr-F, and NH₂-(PEG)ₙ-CO (n=1 to 50)-F, F, K, Orn, C, D, E, 2-aminosuberic acid residue, and Dpr,
X¹⁰ is C or Q,
X¹¹ and X¹² are each independently selected from the group consisting of R, H, D, E, S, T, N, Q, Y, and C, and
X¹³ is C or P.

4. The method according to claim 1 or 2, wherein the Fc-binding IgBP is the following peptide (iii) or (iv):
(iii) a peptide represented by the following formula (I') or (I"):
Z-[(Linker 3)-(X¹₁₋₃)-C-(X²)-(X³)-(X⁴)-(X⁵)-G-(X⁶)-L-(X⁷)-W-C-(X⁸₁₋₃)] ... (I')
[(X¹₁₋₃)-C-(X²)-(X³)-(X⁴)-(X⁵)-G-(X⁶)-L-(X⁷)-W-C-(X⁸₁₋₃)-(Linker 3)]-Z ... (I")
in the formula (I') and the formula (I"),
Z represents a functional group,
(Linker 3) represents a linker,
one to three X¹(S), X², X³, X⁴, X⁵, X⁶, X⁷, and one to three X⁸(s) each mutually independently represent the same or different amino acid residue,
each X¹, X², X³, and each X⁸ mutually independently represent any amino acid residues that are the same or different and are other than C,
X⁴ is H, R, S, or D,
X⁵ is one amino acid residue selected from K, C, D, E, R, V, F, L, 2-aminosuberic acid, Dpr, Orn, AcOrn, AcDab, Dab, Nle, Nva, Tie, Ala(t-Bu), and Cha,
X⁶ is E, N, R, or D, and
X⁷ is I or V; or
(iv) a peptide containing an amino acid sequence represented by the following formula (II'), or a peptide containing an amino acid sequence of (II'), where one or several amino acids are each added, deleted, and/or substituted at a position other than X⁹ to X¹⁴:
X⁹₁₋₂NMQX¹⁰QX¹⁴RFYEALHDPNLNEEQRNAX¹¹IX¹²SIRDDX¹³-(Linker 2)-NH₂ (SEQ ID No. 61) ... (II')
in the formula (II'), (Linker 2) represents a linker, X⁹₁₋₂ is selected from the group consisting of GF, AF, VF, LF, IF, MF, PF, FF, WF, KF, Orn-F, CF, DF, EF, βAla-F, 2-aminosuberic acid-F, Dpr-F, and NH₂-(PEG)ₙ-CO (n=1 to 50)-F, F, K, Orn, C, D, E, a 2-aminosuberic acid residue, Dpr, and Acetyl-K,
X¹⁰ is C or Q,
X¹¹ and X¹² are each independently selected from the group consisting of R, H, D, E, S, T, N, Q, Y, C, and K(Z),
X¹³ is C or P, or absent,
X¹⁴ is R, C, or K(Z), and
Z is a functional group.

5. The method according to any one of claims 1 to 4, wherein the carrier is a column.

6. The method according to any one of claims 1 to 5, wherein a proportion of an Fc molecule where one IgBP is bound to one Fc molecule, relative to the entire Fc molecule, in an Fc molecule composition is 55% or more.

7. A method for preparing a Fc molecule composition comprising a large amount of an IgBP-bound Fc molecule where only one IgG affinity peptide for Fc molecule binding (Fc-binding IgBP) is bound to one molecule having an Fc region (Fc molecule), the method comprising:
reacting an Fc molecule with a carrier to which an IgG affinity peptide for carrier binding (Carrier-binding IgBP) is bound, to thereby obtain an Fc molecule-bound carrier;
reacting an Fc molecule in the Fc molecule-bound carrier obtained, with Fc-binding IgBP, to thereby obtain a bound product of the Fc-binding IgBP and the Fc molecule; and
cutting a bond between the Carrier-binding IgBP and the Fc molecule to recover the bound product of the Fc-binding IgBP and the Fc molecule from the carrier,
wherein the Fc-binding IgBP and the Carrier-binding IgBP are optionally the same or different.

8. The method according to claim 7, wherein the Carrier-binding IgBP is the following peptide (i) or (ii):
(i) a peptide represented by the following formula (I):
NH₂-(Linker)-(X¹₁₋₃)-C-(X²)-(X³)-(X⁴)-(X⁵)-G-(X⁶)-L-(X⁷)-W-C-(X⁸₁₋₃) ... (I)
in the formula (I), (Linker) represents a linker, one to three X¹(s), X², X³, X⁴, X⁵, X⁶, X⁷, and one to three X⁸(s) each mutually independently represent the same or different amino acid residue,
each X¹, X², X³, and each X⁸ mutually independently represent any amino acid residues that are the same or different and are other than C,
X⁴ is H, R, S, or D,
X⁵ is one amino acid residue selected from K, C, D, E, R, V, F, L, 2-aminosuberic acid, Dpr, Orn, AcOrn, AcDab, Dab, Nle, Nva, Tie, Ala(t-Bu), and Cha,
X⁶ is E, N, R, or D, and
X⁷ is I or V; or
(ii) a peptide represented by the following formula (II), or a peptide containing an amino acid sequence of (II), where one or several amino acids are each added, deleted, and/or substituted at a position other than X⁹ to X¹³:
X⁹₁₋₂NMQX¹⁰QRRFYEALHDPNLNEEQRNAX¹¹IX¹²SIRDDX¹³-(Linker 2)-CONH₂ (SEQ ID No. 60) ... (II)
in the formula (II), (Linker 2) represents a linker, X⁹₁₋₂ is selected from the group consisting of GF, AF, VF, LF, IF, MF, PF, FF, WF, KF, Orn-F, CF, DF, EF, βAla-F, 2-aminosuberic acid-F, Dpr-F, and NH₂-(PEG)ₙ-CO (n=1 to 50)-F, F, K, Orn, C, D, E, 2-aminosuberic acid residue, and Dpr,
X¹⁰ is C or Q,
X¹¹ and X¹² are each independently selected from the group consisting of R, H, D, E, S, T, N, Q, Y, and C, and
X¹³ is C or P.

9. The method according to claim 7 or 8, wherein the Fc-binding IgBP is the following peptide (iii) or (iv):
(iii) a peptide represented by the following formula (I') or (I"):
Z-[(Linker 3)-(X¹₁₋₃)-C-(X²)-(X³)-(X⁴)-(X⁵)-G-(X⁶)-L-(X⁷)-W-C-(X⁸₁₋₃)] ... (I')
[(X¹₁₋₃)-C-(X²)-(X³)-(X⁴)-(X⁵)-G-(X⁶)-L-(X⁷)-W-C-(X⁸₁₋₃)-(Linker3)]-Z ... (I")
in the formula (I') and the formula (I"),
Z represents a functional group,
(Linker 3) represents a linker,
one to three X¹(s), X², X³, X⁴, X⁵, X⁶, X⁷, and one to three X⁸(s) each mutually independently represent the same or different amino acid residue,
each X¹, X², X³, and each X⁸ mutually independently represent any amino acid residues that are the same or different and are other than C,
X⁴ is H, R, S, or D,
X⁵ is one amino acid residue selected from K, C, D, E, R, V, F, L, 2-aminosuberic acid, Dpr, Orn, AcOrn, AcDab, Dab, Nle, Nva, Tie, Ala(t-Bu), and Cha,
X⁶ is E, N, R, or D, and
X⁷ is I or V; or
(iv) a peptide containing an amino acid sequence represented by the following formula (II'), or a peptide containing an amino acid sequence of (II'), where one or several amino acids are each added, deleted, and/or substituted at a position other than X⁹ to X¹⁴:
X⁹₁₋₂NMQX¹⁰QX¹⁴RFYEALHDPNLNEEQRNAX¹¹IX¹²SIRDDX¹³-(Linker 2)-NH₂ (SEQ ID No. 61) ... (II')
in the formula (II'), (Linker 2) represents a linker, X⁹₁₋₂ is selected from the group consisting of GF, AF, VF, LF, IF, MF, PF, FF, WF, KF, Orn-F, CF, DF, EF, βAla-F, 2-aminosuberic acid-F, Dpr-F, and NH₂-(PEG)ₙ-CO (n=1 to 50)-F, F, K, Orn, C, D, E, a 2-aminosuberic acid residue, Dpr, and Acetyl-K,
X¹⁰ is C or Q,
X¹¹ and X¹² are each independently selected from the group consisting of R, H, D, E, S, T, N, Q, Y, C, and K(Z),
X¹³ is C or P, or absent,
X¹⁴ is R, C, or K(Z), and
Z is a functional group.

10. The method according to any one of claims 7 to 9, wherein the carrier is a column.

11. The method according to claim 10, wherein the reacting an Fc molecule with a carrier to which Carrier-binding IgBP is bound, to thereby obtain an Fc molecule-bound carrier, is performed by injecting an Fc molecule to a column to which Carrier-binding IgBP is bound, and the reacting the Fc molecule bound to the carrier, with Fc-binding IgBP, to thereby obtain a bound product of the Fc-binding IgBP and the Fc molecule is performed by injecting Fc-binding IgBP, to a column to which an Fc molecule is bound.

12. The method according to claim 11, wherein a proportion of an Fc molecule where one IgBP is bound to one Fc molecule obtained, relative to a total amount of the Fc molecule injected, is 55% or more.

13. The method according to any one of claims 7 to 12, wherein a proportion of an Fc molecule where one IgBP is bound to one Fc molecule, relative to the entire Fc molecule, in the bound product of the Fc-binding IgBP and the Fc molecule, obtained, is 55% or more.

14. A method for preparing an Fc molecule composition comprising a large amount of an Fc molecule (functional group-bound Fc molecule) where a functional group is bound to only one of two Fc chains present in one Fc molecule and where no IgBP is bound, the method comprising:
(i) reacting an Fc molecule with a carrier to which an IgG affinity peptide for carrier binding (Carrier-binding IgBP) is bound, to thereby obtain an Fc molecule-bound carrier;
(ii) reacting an IgG affinity peptide for Fc molecule binding (CCB-IgBP) to which a crosslinking agent is bound via a cleavable linker, with an Fc molecule in the Fc molecule-bound carrier obtained, to thereby obtain a bound product of CCB-IgBP and the Fc molecule;
(iii) reacting and then covalently binding the crosslinking agent and the Fc molecule;
(iv) cleaving the linker to cut a bond between the crosslinking agent and Fc-binding IgBP;
(v) dissociating Fc-binding IgBP, from the Fc molecule, to thereby obtain a functional group-bound Fc molecule bound to the carrier, and free Fc-binding IgBP; and
(vi) recovering the functional group-bound Fc molecule from the carrier, wherein
the Fc-binding IgBP and the Carrier-binding IgBP are optionally the same or different,
a group containing a functional group is optionally present between the cleavable linker and the crosslinking agent, and
the functional group contained in the functional group-bound Fc molecule is a functional group present between the cleavable linker and the crosslinking agent, or a group generated by linker cleavage.

15. A method for preparing an Fc molecule composition comprising a large amount of an Fc molecule (functional molecule-bound Fc molecule) where a functional molecule is bound to only one of two Fc chains present in one Fc molecule and where no IgBP is bound, the method comprising:
(i) reacting an Fc molecule with a carrier to which an IgG affinity peptide for carrier binding (Carrier-binding IgBP) is bound, to thereby obtain an Fc molecule-bound carrier;
(ii) reacting an IgG affinity peptide for Fc molecule binding (CCB-IgBP) to which a crosslinking agent is bound via a cleavable linker, with an Fc molecule in the Fc molecule-bound carrier obtained, to thereby obtain a bound product of CCB-IgBP and the Fc molecule;
(iii) reacting and then covalently binding the crosslinking agent and the Fc molecule;
(iv) cleaving the linker to cut a bond between the crosslinking agent and Fc-binding IgBP;
(v) dissociating Fc-binding IgBP, from the Fc molecule, to thereby obtain a functional group-bound Fc molecule bound to the carrier, and free Fc-binding IgBP;
(vi) binding a functional molecule to the functional group of the functional group-bound Fc molecule to thereby obtain a functional molecule-bound Fc molecule; and
(vii) recovering the functional molecule-bound Fc molecule from the carrier,
wherein
the Fc-binding IgBP and the Carrier-binding IgBP are optionally the same or different,
a group containing a functional group is optionally present between the cleavable linker and the crosslinking agent, and
the functional group contained in the functional group-bound Fc molecule is a functional group present between the cleavable linker and the crosslinking agent, or a group generated by linker cleavage.

16. A method for preparing an Fc molecule composition comprising a large amount of a functional molecule-bound Fc molecule where a functional molecule is bound to only one of two Fc chains present in one Fc molecule and where no IgBP is bound, the method comprising:
(i) reacting an Fc molecule with a carrier to which an IgG affinity peptide for carrier binding (Carrier-binding IgBP) is bound, to thereby obtain an Fc molecule-bound carrier;
(ii) reacting an IgG affinity peptide for Fc molecule binding (CCB-IgBP) to which a crosslinking agent is bound via a cleavable linker, with an Fc molecule in the Fc molecule-bound carrier obtained, to thereby obtain a bound product of CCB-IgBP and the Fc molecule;
(iii) reacting and then covalently binding the crosslinking agent and the Fc molecule;
(iv) cleaving the linker to cut a bond between the crosslinking agent and Fc-binding IgBP;
(v) dissociating Fc-binding IgBP, from the Fc molecule, to thereby obtain a functional molecule-bound Fc molecule bound to the carrier via Carrier-binding IgBP, and free Fc-binding IgBP; and
(vi) recovering the functional molecule-bound Fc molecule from the carrier,
wherein
the Fc-binding IgBP and the Carrier-binding IgBP are optionally the same or different, and
a functional molecule is bound between the cleavable linker and the crosslinking agent.

17. The method according to any one of claims 14 to 16, wherein the carrier is a column.

18. The method according to any one of claims 14 to 17, wherein the method further comprises, after the cleaving the linker to cut a bond between the crosslinking agent and the IgG affinity peptide for Fc molecule binding (Fc-binding IgBP) and before the cutting a bond between the Carrier-binding IgBP and the Fc molecule to recover a functional group-bound Fc molecule from the carrier, binding another functional molecule to the functional group.

19. An Fc molecule composition, wherein any one proportion of the following (a) to (c) is higher than such any one proportion obtained in synthesis in a test tube according to a CCAP method:
(a) a proportion of a molecule (Fc molecule) having an Fc region of IgG, to which only one IgG affinity peptide (IgBP) to be specifically bound to an Fc region of IgG is bound, relative to the entire Fc molecule;
(b) a proportion of a functional group-bound Fc molecule where a functional group is bound to only one of two Fc chains present in an Fc region-containing one molecule (Fc molecule), relative to the entire Fc molecule; or
(c) a proportion of a functional group-bound Fc molecule where another functional molecule is bound to only one of two Fc chains present in an Fc region-containing one molecule (Fc molecule), relative to the entire Fc molecule.

20. An Fc molecule composition, wherein any one proportion of the following (a) to (c) is 55% or more:
(a) a proportion of a molecule (Fc molecule) having an Fc region of IgG, to which only one IgG affinity peptide (IgBP) to be specifically bound to an Fc region of IgG is bound, in the entire Fc molecule;
(b) a proportion of a functional group-bound Fc molecule where a functional group is bound to only one of two Fc chains present in an Fc region-containing one molecule (Fc molecule), relative to the entire Fc molecule; or
(c) a proportion of a functional group-bound Fc molecule where another functional molecule is bound to only one of two Fc chains present in an Fc region-containing one molecule (Fc molecule), relative to the entire Fc molecule.

21. The composition according to claim 19 or 20, wherein the Fc molecule is IgG or Fc fusion protein.

22. The composition according to any one of claims 19 to 21, wherein the IgBP is the following peptide (iii) or (iv) to be specifically bound to an Fc region of IgG, the peptides (iii) and (iv) being the same or different:
(iii) a peptide represented by the following formula (I') or (I"):
Z-[(Linker 3)-(X¹₁₋₃)-C-(X²)-(X³)-(X⁴)-(X⁵)-G-(X⁶)-L-(X⁷)-W-C-(X⁸₁₋₃)] ... (I')
[(X¹₁₋₃)-C-(X²)-(X³)-(X⁴)-(X⁵)-G-(X⁶)-L-(X⁷)-W-C-(X⁸₁₋₃)-(Linker 3)]-Z ... (I")
in the formula (I') and the formula (I"),
Z represents a functional group,
(Linker 3) represents a linker,
one to three X¹(s), X², X³, X⁴, X⁵, X⁶, X⁷, and one to three X⁸(s) each mutually independently represent the same or different amino acid residue,
each X¹, X², X³, and each X⁸ mutually independently represent any amino acid residues that are the same or different and are other than C,
X⁴ is H, R, S, or D,
X⁵ is one amino acid residue selected from K, C, D, E, R, V, F, L, 2-aminosuberic acid, Dpr, Orn, AcOrn, AcDab, Dab, Nle, Nva, Tie, Ala(t-Bu), and Cha,
X⁶ is E, N, R, or D, and
X⁷ is I or V; or
(iv) a peptide containing an amino acid sequence represented by the following formula (II'), or a peptide containing an amino acid sequence of (II'), where one or several amino acids are each added, deleted, and/or substituted at a position other than X⁹ to X¹⁴:
X⁹₁₋₂NMQX¹⁰QX¹⁴RFYEALHDPNLNEEQRNAX¹¹IX¹²SIRDDX¹³-(Linker 2)-NH₂ (SEQ ID No. 61) ... (II')
in the formula (II'), (Linker 2) represents a linker, X⁹₁₋₂ is selected from the group consisting of GF, AF, VF, LF, IF, MF, PF, FF, WF, KF, Orn-F, CF, DF, EF, βAla-F, 2-aminosuberic acid-F, Dpr-F, and NH₂-(PEG)ₙ-CO (n=1 to 50)-F, F, K, Orn, C, D, E, a 2-aminosuberic acid residue, Dpr, and Acetyl-K,
X¹⁰ is C or Q,
X¹¹ and X¹² are each independently selected from the group consisting of R, H, D, E, S, T, N, Q, Y, C, and K(Z),
X¹³ is C or P, or absent,
X¹⁴ is R, C, or K(Z), and
Z is a functional group.

23. The composition according to any one of claims 19 to 22, wherein the composition is a medical or diagnostic composition.

24. A carrier to which the following peptide (i) or (ii) is bound:
(i) a peptide represented by the following formula (I):
NH₂-(Linker)-(X¹₁₋₃)-C-(X²)-(X³)-(X⁴)-(X⁵)-G-(X⁶)-L-(X⁷)-W-C-(X⁸₁₋₃) ... (I)
in the formula (I), (Linker) represents a linker, one to three X¹(s), X², X³, X⁴, X⁵, X⁶, X⁷, and one to three X⁸(s) each mutually independently represent the same or different amino acid residue,
each X¹, X², X³, and each X⁸ mutually independently represent any amino acid residues that are the same or different and are other than C,
X⁴ is H, R, S, or D,
X⁵ is one amino acid residue selected from K, C, D, E, R, V, F, L, 2-aminosuberic acid, Dpr, Orn, AcOrn, AcDab, Dab, Nle, Nva, Tie, Ala(t-Bu), and Cha,
X⁶ is E, N, R, or D, and
X⁷ is I or V; or
(ii) a peptide represented by the following formula (II), or a peptide containing an amino acid sequence of (II), where one or several amino acids are each added, deleted, and/or substituted at a position other than X⁹ to X¹³:
X⁹₁₋₂NMQX¹⁰QRRFYEALHDPNLNEEQRNAX¹¹IX¹²SIRDDX¹³-(Linker 2)-CONH₂ (SEQ ID No. 60) ... (II)
in the formula (II), (Linker 2) represents a linker, X⁹₁₋₂ is selected from the group consisting of GF, AF, VF, LF, IF, MF, PF, FF, WF, KF, Orn-F, CF, DF, EF, βAla-F, 2-aminosuberic acid-F, Dpr-F, and NH₂-(PEG)ₙ-CO (n=1 to 50)-F, F, K, Orn, C, D, E, 2-aminosuberic acid residue, and Dpr,
X¹⁰ is C or Q,
X¹¹ and X¹² are each independently selected from the group consisting of R, H, D, E, S, T, N, Q, Y, and C, and
X¹³ is C or P.


SEQUENCE LISTING

<110>  Kagoshima University

<120>  Separation and purification of CCAP product using afinity column

<130>  KGU-6-PCT

<150>  JP2019-193830
<151>  2019-10-24

<160>  72

<170>  PatentIn version 3.5

<210>  1
<211>  13
<212>  PRT
<213>  Artificial Sequence

<220>
<223>  IgG-BP



<220>
<221>  MISC_FEATURE
<222>  (6)..(6)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  1

Asp Cys Ala Tyr His Xaa Gly Glu Leu Val Trp Cys Thr
1               5                   10


<210>  2
<211>  17
<212>  PRT
<213>  Artificial Sequence

<220>
<223>  IgG-BP



<220>
<221>  MISC_FEATURE
<222>  (8)..(8)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  2

Gly Pro Asp Cys Ala Tyr His Xaa Gly Glu Leu Val Trp Cys Thr Phe
1               5                   10                  15


His



<210>  3
<211>  13

<212>  PRT
<213>  Artificial Sequence

<220>
<223>  IgG-BP


<220>
<221>  MISC_FEATURE
<222>  (6)..(6)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  3

Arg Cys Ala Tyr His Xaa Gly Glu Leu Val Trp Cys Ser
1               5                   10


<210>  4
<211>  17
<212>  PRT
<213>  Artificial Sequence

<220>
<223>  IgG-BP


<220>
<221>  MISC_FEATURE
<222>  (8)..(8)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  4

Gly Pro Arg Cys Ala Tyr His Xaa Gly Glu Leu Val Trp Cys Ser Phe
1               5                   10                  15


His




<210>  5
<211>  17
<212>  PRT
<213>  Artificial Sequence

<220>
<223>  IgG-BP


<220>
<221>  MISC_FEATURE
<222>  (8)..(8)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  5

Ser Pro Asp Cys Ala Tyr His Xaa Gly Glu Leu Val Trp Cys Thr Phe
1               5                   10                  15

His

<210>  6
<211>  17
<212>  PRT
<213>  Artificial Sequence

<220>
<223>  IgG-BP

<220>
<221>  MISC_FEATURE
<222>  (8)..(8)
<223>  X is K,C,D,E, R, 2-Aminosuberic acid, or Diaminopropionic acid

<400>  6

Gly Asp Asp Cys Ala Tyr His Xaa Gly Glu Leu Val Trp Cys Thr Phe
1               5                   10                  15

His

<210>  7
<211>  17
<212>  PRT
<213>  Artificial Sequence

<220>
<223>  IgG-BP

<220>
<221>  MISC_FEATURE
<222>  (8)..(8)
<223>  X is K, C, D, E, R, V, F, L, 2-Aminosuberic acid, Dpr, Orn,
        AcOrn, AcDab, Dab, Nle, Nva, Tle, Ala(t-Bu), or Cha

<400>  7

Gly Pro Ser Cys Ala Tyr His Xaa Gly Glu Leu Val Trp Cys Thr Phe
1               5                   10                  15

His

<210>  8
<211>  17
<212>  PRT
<213>  Artificial Sequence

<220>
<223>  IgG-BP

<220>
<221>  MISC_FEATURE
<222>  (8)..(8)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  8

Gly Pro Asp Cys Ala Tyr His Xaa Gly Glu Leu Val Trp Cys Ser Phe
1               5                   10                  15

His

<210>  9
<211>  17
<212>  PRT
<213>  Artificial Sequence

<220>
<223>  IgG-BP

<220>
<221>  MISC_FEATURE
<222>  (8)..(8)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  9

Gly Pro Asp Cys Ala Tyr His Xaa Gly Glu Leu Val Trp Cys Thr His
1               5                   10                  15

His

<210>  10
<211>  17
<212>  PRT
<213>  Artificial Sequence

<220>
<223>  IgG-BP

<220>
<221>  MISC_FEATURE
<222>  (8)..(8)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  10

Gly Pro Asp Cys Ala Tyr His Xaa Gly Glu Leu Val Trp Cys Thr Phe
1               5                   10                  15

Tyr

<210>  11
<211>  17
<212>  PRT
<213>  Artificial Sequence

<220>
<223>  IgG-BP

<220>
<221>  MISC_FEATURE
<222>  (8)..(8)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  11

Ser Pro Asp Cys Ala Tyr His Xaa Gly Glu Leu Val Trp Cys Thr Phe
1               5                   10                  15

Tyr

<210>  12
<211>  17
<212>  PRT
<213>  Artificial Sequence

<220>
<223>  IgG-BP

<220>
<221>  MISC_FEATURE
<222>  (8)..(8)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  12

Ser Asp Asp Cys Ala Tyr His Xaa Gly Glu Leu Val Trp Cys Thr Phe
1               5                   10                  15

Tyr

<210>  13
<211>  17
<212>  PRT
<213>  Artificial Sequence

<220>
<223>  IgG-BP

<220>
<221>  MISC_FEATURE
<222>  (8)..(8)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  13

Arg Gly Asn Cys Ala Tyr His Xaa Gly Gln Leu Val Trp Cys Thr Tyr
1               5                   10                  15

His

<210>  14
<211>  17
<212>  PRT
<213>  Artificial Sequence

<220>
<223>  IgG-BP

<220>
<221>  MISC_FEATURE
<222>  (2)..(2)
<223>  X is homocysteine

<220>
<221>  MISC_FEATURE
<222>  (8)..(8)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<220>
<221>  misc_feature
<222>  (16)..(16)
<223>  Xaa can be any naturally occurring amino acid

<220>
<221>  MISC_FEATURE
<222>  (17)..(17)
<223>  X is homocysteine

<400>  14

Gly Xaa Asp Cys Ala Tyr His Xaa Gly Glu Leu Val Trp Cys Thr Xaa
1               5                   10                  15

His

<210>  15
<211>  19
<212>  PRT
<213>  Artificial Sequence

<220>
<223>  IgG-BP

<220>
<221>  MISC_FEATURE
<222>  (10)..(10)
<223>  X is K, C, D, E, R, V, F, L, 2-Aminosuberic acid, Dpr, Orn,
      AcOrn, AcDab, Dab, Nle, Nva, Tle, Ala(t-Bu), or Cha

<400>  15

Arg Arg Gly Pro Asp Cys Ala Tyr His Xaa Gly Glu Leu Val Trp Cys
1               5                   10                  15

Thr Phe His

<210>  16
<211>  13
<212>  PRT
<213>  Artificial Sequence

<220>
<223>  IgG-BP

<220>
<221>  MISC_FEATURE
<222>  (6)..(6)
<223>  X is K, C, D, E, R, V, F, L, 2-Aminosuberic acid, Dpr, Orn,
      AcOrn, AcDab, Dab, Nle, Nva, Tle, Ala(t-Bu), or Cha

<400>  16

Asp Cys Thr Tyr His Xaa Gly Asn Leu Val Trp Cys Thr
1               5                   10

<210>  17
<211>  13
<212>  PRT
<213>  Artificial Sequence

<220>
<223>  IgG-BP

<220>
<221>  MISC_FEATURE
<222>  (6)..(6)
<223>  X is K, C, D, E, R, V, F, L, 2-Aminosuberic acid, Dpr, Orn,
      AcOrn, AcDab, Dab, Nle, Nva, Tle, Ala(t-Bu), or Cha

<400>  17

Asp Cys Ala Tyr His Xaa Gly Asn Leu Val Trp Cys Thr
1               5                   10

<210>  18
<211>  13
<212>  PRT

<213>  Artificial Sequence

<220>
<223>  IgG-BP


<220>
<221>  MISC_FEATURE
<222>  (6)..(6)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  18

Asp Cys Thr Tyr His Xaa Gly Glu Leu Val Trp Cys Thr
1               5                   10


<210>  19
<211>  13
<212>  PRT
<213>  Artificial Sequence

<220>
<223>  IgG-BP


<220>
<221>  MISC_FEATURE
<222>  (6)..(6)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  19

Asp Cys Ala Trp His Xaa Gly Glu Leu Val Trp Cys Thr
1               5                   10


<210>  20
<211>  13
<212>  PRT
<213>  Artificial Sequence

<220>
<223>  IgG-BP


<220>
<221>  MISC_FEATURE
<222>  (6)..(6)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  20

Asp Cys Thr Tyr Thr Xaa Gly Asn Leu Val Trp Cys Thr
1               5                   10


<210>  21
<211>  13
<212>  PRT

<213>  Artificial Sequence

<220>
<223>  IgG-BP


<220>
<221>  MISC_FEATURE
<222>  (6)..(6)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  21

Asp Cys Ala Tyr Thr Xaa Gly Asn Leu Val Trp Cys Thr
1               5                   10


<210>  22
<211>  13
<212>  PRT
<213>  Artificial Sequence

<220>
<223>  IgG-BP


<220>
<221>  MISC_FEATURE
<222>  (6)..(6)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  22

Asp Cys Ser Tyr Thr Xaa Gly Asn Leu Val Trp Cys Thr
1               5                   10


<210>  23
<211>  13
<212>  PRT
<213>  Artificial Sequence

<220>
<223>  IgG-BP


<220>
<221>  MISC_FEATURE
<222>  (6)..(6)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  23

Asp Cys Thr Trp Thr Xaa Gly Asn Leu Val Trp Cys Thr
1               5                   10


<210>  24
<211>  13
<212>  PRT

<213>  Artificial Sequence

<220>
<223>  IgG-BP


<220>
<221>  MISC_FEATURE
<222>  (6)..(6)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  24

Asp Cys Thr Tyr His Xaa Gly Asn Leu Val Trp Cys Thr
1               5                   10


<210>  25
<211>  13
<212>  PRT
<213>  Artificial Sequence

<220>
<223>  IgG-BP


<220>
<221>  MISC_FEATURE
<222>  (6)..(6)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  25

Asp Cys Thr Tyr Arg Xaa Gly Asn Leu Val Trp Cys Thr
1               5                   10


<210>  26
<211>  13
<212>  PRT
<213>  Artificial Sequence

<220>
<223>  IgG-BP


<220>
<221>  MISC_FEATURE
<222>  (6)..(6)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  26

Asp Cys Thr Tyr Ser Xaa Gly Asn Leu Val Trp Cys Thr
1               5                   10


<210>  27
<211>  13
<212>  PRT

<213>  Artificial Sequence

<220>
<223>  IgG-BP


<220>
<221>  MISC_FEATURE
<222>  (6)..(6)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  27

Asp Cys Thr Tyr Thr Xaa Gly Asn Leu Val Trp Cys Thr
1               5                   10


<210>  28
<211>  13
<212>  PRT
<213>  Artificial Sequence

<220>
<223>  IgG-BP


<220>
<221>  MISC_FEATURE
<222>  (6)..(6)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  28

Asp Cys Thr Tyr Thr Xaa Gly Glu Leu Val Trp Cys Thr
1               5                   10


<210>  29
<211>  13
<212>  PRT
<213>  Artificial Sequence

<220>
<223>  IgG-BP


<220>
<221>  MISC_FEATURE
<222>  (6)..(6)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  29

Asp Cys Thr Tyr Thr Xaa Gly Arg Leu Val Trp Cys Thr
1               5                   10


<210>  30
<211>  13
<212>  PRT

<213>  Artificial Sequence

<220>
<223>  IgG-BP


<220>
<221>  MISC_FEATURE
<222>  (6)..(6)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  30

Asp Cys Thr Tyr Thr Xaa Gly Asp Leu Val Trp Cys Thr
1               5                   10


<210>  31
<211>  13
<212>  PRT
<213>  Artificial Sequence

<220>
<223>  IgG-BP


<220>
<221>  MISC_FEATURE
<222>  (6)..(6)
<223>  X is K, C, D, E, R, V, F, L, 2-Aminosuberic acid, Dpr, Orn,
       AcOrn, AcDab, Dab, Nle, Nva, Tle, Ala(t-Bu), or Cha

<400>  31

Asp Cys Thr Tyr Thr Xaa Gly Asn Leu Ile Trp Cys Thr
1               5                   10


<210>  32
<211>  13
<212>  PRT
<213>  Artificial Sequence

<220>
<223>  IgG-BP

<400>  32

Asp Cys Ala Tyr His Arg Gly Glu Leu Val Trp Cys Thr
1               5                   10


<210>  33
<211>  17
<212>  PRT
<213>  Artificial Sequence

<220>
<223>  IgG-BP

<400>  33

Gly Pro Asp Cys Ala Tyr His Arg Gly Glu Leu Val Trp Cys Thr Phe
1               5                   10                  15

His

<210>  34
<211>  13
<212>  PRT
<213>  Artificial Sequence

<220>
<223>  IgG-BP

<400>  34

Arg Cys Ala Tyr His Arg Gly Glu Leu Val Trp Cys Ser
1               5                   10

<210>  35
<211>  17
<212>  PRT
<213>  Artificial Sequence

<220>
<223>  IgG-BP

<400>  35

Gly Pro Arg Cys Ala Tyr His Arg Gly Glu Leu Val Trp Cys Ser Phe
1               5                   10                  15

His

<210>  36
<211>  17
<212>  PRT
<213>  Artificial Sequence

<220>
<223>  IgG-BP

<400>  36

Ser Pro Asp Cys Ala Tyr His Arg Gly Glu Leu Val Trp Cys Thr Phe
1               5                   10                  15

His

<210>  37
<211>  17
<212>  PRT
<213>  Artificial Sequence

<220>
<223>  IgG-BP

<400>  37

Gly Asp Asp Cys Ala Tyr His Arg Gly Glu Leu Val Trp Cys Thr Phe
1               5                   10                  15

His

<210>  38
<211>  17
<212>  PRT
<213>  Artificial Sequence

<220>
<223>  IgG-BP

<400>  38

Gly Pro Ser Cys Ala Tyr His Arg Gly Glu Leu Val Trp Cys Thr Phe
1               5                   10                  15

His

<210>  39
<211>  17
<212>  PRT
<213>  Artificial Sequence

<220>
<223>  IgG-BP

<400>  39

Gly Pro Asp Cys Ala Tyr His Arg Gly Glu Leu Val Trp Cys Ser Phe
1               5                   10                  15

His

<210>  40
<211>  17
<212>  PRT
<213>  Artificial Sequence

<220>
<223>  IgG-BP

<400>  40

Gly Pro Asp Cys Ala Tyr His Arg Gly Glu Leu Val Trp Cys Thr His
1               5                   10                  15

His

<210>  41
<211>  17
<212>  PRT
<213>  Artificial Sequence

<220>
<223>  IgG-BP

<400>  41

Gly Pro Asp Cys Ala Tyr His Arg Gly Glu Leu Val Trp Cys Thr Phe
1               5                   10                  15

Tyr

<210>  42
<211>  17
<212>  PRT
<213>  Artificial Sequence

<220>
<223>  IgG-BP

<400>  42

Ser Pro Asp Cys Ala Tyr His Arg Gly Glu Leu Val Trp Cys Thr Phe
1               5                   10                  15

Tyr

<210>  43
<211>  17
<212>  PRT
<213>  Artificial Sequence

<220>
<223>  IgG-BP

<400>  43

Ser Asp Asp Cys Ala Tyr His Arg Gly Glu Leu Val Trp Cys Thr Phe
1               5                   10                  15

Tyr

<210>  44
<211>  13
<212>  PRT
<213>  Artificial Sequence

<220>
<223>  IgG-BP

<400>  44

Asp Cys Thr Tyr His Arg Gly Asn Leu Val Trp Cys Thr
1               5                   10


<210>  45
<211>  13
<212>  PRT
<213>  Artificial Sequence

<220>
<223>  IgG-BP

<400>  45

Asp Cys Ala Tyr His Arg Gly Asn Leu Val Trp Cys Thr
1               5                   10


<210>  46
<211>  13
<212>  PRT
<213>  Artificial Sequence

<220>
<223>  IgG-BP

<400>  46

Asp Cys Thr Tyr His Arg Gly Glu Leu Val Trp Cys Thr
1               5                   10


<210>  47
<211>  13
<212>  PRT
<213>  Artificial Sequence

<220>
<223>  IgG-BP

<400>  47

Asp Cys Ala Trp His Arg Gly Glu Leu Val Trp Cys Thr
1               5                   10


<210>  48
<211>  13
<212>  PRT
<213>  Artificial Sequence

<220>
<223>  IgG-BP

<400>  48

Asp Cys Thr Tyr Thr Asn Gly Asn Leu Val Trp Cys Thr
1               5                   10

<210>  49
<211>  13
<212>  PRT
<213>  Artificial Sequence

<220>
<223>  IgG-BP

<400>  49

Asp Cys Ala Tyr Thr Asn Gly Asn Leu Val Trp Cys Thr
1               5                   10


<210>  50
<211>  13
<212>  PRT
<213>  Artificial Sequence

<220>
<223>  IgG-BP

<400>  50

Asp Cys Ser Tyr Thr Asn Gly Asn Leu Val Trp Cys Thr
1               5                   10


<210>  51
<211>  13
<212>  PRT
<213>  Artificial Sequence

<220>
<223>  IgG-BP

<400>  51

Asp Cys Thr Trp Thr Asn Gly Asn Leu Val Trp Cys Thr
1               5                   10


<210>  52
<211>  13
<212>  PRT
<213>  Artificial Sequence

<220>
<223>  IgG-BP

<400>  52

Asp Cys Thr Tyr His Asn Gly Asn Leu Val Trp Cys Thr
1               5                   10


<210>  53
<211>  13
<212>  PRT
<213>  Artificial Sequence

<220>
<223>  IgG-BP

<400>  53

Asp Cys Thr Tyr Arg Asn Gly Asn Leu Val Trp Cys Thr
1               5                   10


<210>  54
<211>  13
<212>  PRT
<213>  Artificial Sequence

<220>
<223>  IgG-BP

<400>  54

Asp Cys Thr Tyr Ser Asn Gly Asn Leu Val Trp Cys Thr
1               5                   10


<210>  55
<211>  13
<212>  PRT
<213>  Artificial Sequence

<220>
<223>  IgG-BP

<400>  55

Asp Cys Thr Tyr Thr Arg Gly Asn Leu Val Trp Cys Thr
1               5                   10


<210>  56
<211>  13
<212>  PRT
<213>  Artificial Sequence

<220>
<223>  IgG-BP

<400>  56

Asp Cys Thr Tyr Thr Asn Gly Glu Leu Val Trp Cys Thr
1               5                   10


<210>  57
<211>  13
<212>  PRT
<213>  Artificial Sequence

<220>
<223>  IgG-BP

<400>  57

Asp Cys Thr Tyr Thr Asn Gly Arg Leu Val Trp Cys Thr
1               5                   10

<210>  58
<211>  13
<212>  PRT
<213>  Artificial Sequence

<220>
<223>  IgG-BP

<400>  58

Asp Cys Thr Tyr Thr Asn Gly Asp Leu Val Trp Cys Thr
1               5                   10


<210>  59
<211>  13
<212>  PRT
<213>  Artificial Sequence

<220>
<223>  IgG-BP

<400>  59

Asp Cys Thr Tyr Thr Asn Gly Asn Leu Ile Trp Cys Thr
1               5                   10


<210>  60
<211>  35
<212>  PRT
<213>  Artificial Sequence

<220>
<223>  IgG-BP


<220>
<221>  MISC_FEATURE
<222>  (1)..(1)
<223>  X is G, A, V, L, I, M, P, F, W, K, Orn, C, D, E, beta-Ala,
       2-Aminosuberic acid, Diaminopropionic acid, or None

<220>
<221>  MISC_FEATURE
<222>  (2)..(2)
<223>  X is F, K, Orn, C, D, E, 2-Aminosuberic acid, Diaminopropionic
       acid, or Acetyl-K(K, Orn, C, D, E, 2-Aminosuberic acid,
       Diaminopropionic acid, or Acetyl-K are selectable only when 1st X
       is None)

<220>
<221>  MISC_FEATURE
<222>  (6)..(6)
<223>  X is C or Q

<220>
<221>  MISC_FEATURE
<222>  (27)..(27)
<223>  X is R, H, D, E, S, T, N, Q, Y, or C

<220>
<221>  MISC_FEATURE
<222>  (29)..(29)
<223>  X is R, H, D, E, S, T, N, Q, Y, or C

<220>
<221>  MISC_FEATURE
<222>  (35)..(35)
<223>  X is C, P, or None

<400>  60

Xaa Xaa Asn Met Gln Xaa Gln Arg Arg Phe Tyr Glu Ala Leu His Asp
1               5                   10                  15

Pro Asn Leu Asn Glu Glu Gln Arg Asn Ala Xaa Ile Xaa Ser Ile Arg
            20                  25                  30

Asp Asp Xaa
        35

<210>  61
<211>  35
<212>  PRT
<213>  Artificial Sequence

<220>
<223>  IgG-BP

<220>
<221>  MISC_FEATURE
<222>  (1)..(1)
<223>  X is G, A, V, L, I, M, P, F, W, K, Orn, C, D, E, beta-Ala,
       2-Aminosuberic acid, Diaminopropionic acid, or None

<220>
<221>  MISC_FEATURE
<222>  (2)..(2)
<223>  X is F, K, Orn, C, D, E, 2-Aminosuberic acid, Diaminopropionic
       acid, or Acetyl-K(K, Orn, C, D, E, 2-Aminosuberic acid,
       Diaminopropionic acid, or Acetyl-K are selectable only when 1st X
       is None)

<220>
<221>  MISC_FEATURE
<222>  (6)..(6)
<223>  X is C or Q

<220>
<221>  MISC_FEATURE
<222>  (8)..(8)
<223>  X is R, C, K, or K(Z) (Z is a functional group)

<220>
<221>  MISC_FEATURE
<222>  (27)..(27)
<223>  X is R, H, D, E, S, T, N, Q, Y, C, or K(Z) (Z is a functional
       group)

<220>
<221>  MISC_FEATURE
<222>  (29)..(29)
<223>  X is R, H, D, E, S, T, N, Q, Y, C, or K(Z) (Z is a functional
      group)

<220>
<221>  MISC_FEATURE
<222>  (35)..(35)
<223>  X is C, P, or None

<400>  61

Xaa Xaa Asn Met Gln Xaa Gln Xaa Arg Phe Tyr Glu Ala Leu His Asp
1               5                   10                  15

Pro Asn Leu Asn Glu Glu Gln Arg Asn Ala Xaa Ile Xaa Ser Ile Arg
            20                  25                  30

Asp Asp Xaa
        35

<210>  62
<211>  34
<212>  PRT
<213>  Artificial Sequence

<220>
<223>  IgG-BP

<400>  62

Phe Asn Met Gln Cys Gln Arg Arg Phe Tyr Glu Ala Leu His Asp Pro
1               5                   10                  15

Asn Leu Asn Glu Glu Gln Arg Asn Ala Arg Ile Arg Ser Ile Arg Asp
            20                  25                  30

Asp Cys

<210>  63
<211>  35
<212>  PRT
<213>  Artificial Sequence

<220>
<223>  IgG-BP

<400>  63

Gly Phe Asn Met Gln Cys Gln Arg Arg Phe Tyr Glu Ala Leu His Asp
1               5                   10                  15

Pro Asn Leu Asn Glu Glu Gln Arg Asn Ala Arg Ile Arg Ser Ile Arg
            20                  25                  30

Asp Asp Cys
        35

<210>  64
<211>  34
<212>  PRT
<213>  Artificial Sequence

<220>
<223>  IgG-BP

<400>  64

Lys Asn Met Gln Cys Gln Arg Arg Phe Tyr Glu Ala Leu His Asp Pro
1               5                   10                  15

Asn Leu Asn Glu Glu Gln Arg Asn Ala Arg Ile Arg Ser Ile Arg Asp
            20                  25                  30

Asp Cys

<210>  65
<211>  35
<212>  PRT
<213>  Artificial Sequence

<220>
<223>  IgG-BP

<400>  65

Gly Phe Asn Met Gln Cys Gln Lys Arg Phe Tyr Glu Ala Leu His Asp
1               5                   10                  15

Pro Asn Leu Asn Glu Glu Gln Arg Asn Ala Arg Ile Arg Ser Ile Arg
            20                  25                  30

Asp Asp Cys
        35

<210>  66
<211>  34
<212>  PRT
<213>  Artificial Sequence

<220>
<223>  IgG-BP

<400>  66

Lys Asn Met Gln Cys Gln Lys Arg Phe Tyr Glu Ala Leu His Asp Pro
1               5                   10                  15

Asn Leu Asn Glu Glu Gln Arg Asn Ala Arg Ile Arg Ser Ile Arg Asp
            20                  25                  30

Asp Cys

<210>  67
<211>  33
<212>  PRT
<213>  Artificial Sequence

<220>
<223>  IgG-BP

<400>  67

Phe Asn Met Gln Gln Gln Arg Arg Phe Tyr Glu Ala Leu His Asp Pro
1               5                   10                  15

Asn Leu Asn Glu Glu Gln Arg Asn Ala Arg Ile Arg Ser Ile Arg Asp
            20                  25                  30

Asp

<210>  68
<211>  35
<212>  PRT
<213>  Artificial Sequence

<220>
<223>  IgG-BP

<400>  68

Gly Lys Asn Met Gln Cys Gln Arg Arg Phe Tyr Glu Ala Leu His Asp
1               5                   10                  15

Pro Asn Leu Asn Glu Glu Gln Arg Asn Ala Arg Ile Arg Ser Ile Arg
            20                  25                  30

Asp Asp Cys
        35

<210>  69
<211>  19
<212>  PRT
<213>  Artificial Sequence

<220>
<223>  IgG-BP

<220>

<221>  MISC_FEATURE
<222>  (10)..(10)
<223>  K is modified with succinimidyl glutaryl group

<400>  69

Glu Glu Gly Pro Asp Cys Ala Tyr His Lys Gly Glu Leu Val Trp Cys
1               5                   10                  15

Thr Phe His

<210>  70
<211>  17
<212>  PRT
<213>  Artificial Sequence

<220>
<223>  IgG-BP

<400>  70

Gly Ser Gly Gly Ser Asp Cys Ala Tyr His Arg Gly Glu Leu Val Trp
1               5                   10                  15

Cys

<210>  71
<211>  40
<212>  PRT
<213>  Artificial Sequence

<220>
<223>  IgG-BP

<400>  71

Phe Asn Met Gln Gln Gln Arg Arg Phe Tyr Glu Ala Leu His Asp Pro
1               5                   10                  15

Asn Leu Asn Glu Glu Gln Arg Asn Ala Arg Ile Arg Ser Ile Arg Asp
            20                  25                  30

Asp Pro Ser Gly Ser Gly Ser Lys
        35                  40

<210>  72
<211>  15
<212>  PRT
<213>  Artificial Sequence

<220>
<223>  IgG-BP

<400>  72

Arg Arg Asp Cys Ala Tyr His Lys Gly Glu Leu Val Trp Cys Thr
1               5                   10                  15